# FASCICULE DE BREVET EUROPEEN

(11) **EP 3 541 517 B1**
(45) Date de publication et mention de la délivrance du brevet: **22.05.2024**
(21) Numéro de dépôt: 17811989.7
(22) Date de dépôt: 15.11.2017
(51) Int. Cl.: B01L 3/00, G01N 1/40

(54) **SYSTÈME ET PROCÉDÉ D'EXTRACTION POUR EXTRAIRE DES MICRO-ORGANISMES CONTENUS DANS UN ÉCHANTILLON**
EXTRAKTIONSSYSTEM UND VERFAHREN ZUM EXTRAHIEREN VON IN EINER PROBE ENTHALTENEN MIKROORGANISMEN
EXTRACTION SYSTEM AND METHOD FOR EXTRACTING MICROORGANISMS CONTAINED IN A SAMPLE

(30) Priorité: 15.11.2016 FR 1661015
(43) Date de publication de la demande: 25.09.2019
(73) Titulaire: bioMérieux, 69280 Marcy-l'Étoile (FR)
(72) Inventeur: BROYER, Patrick, 38500 Saint Cassien (FR); CHARLES, Marie-Hélène, 69420 Condrieu (FR); BLAZE, Jérôme, 38660 La Terrasse (FR); PERROT, Nadine, 01120 Montluel (FR); PINSTON, Frédéric, 38000 Grenoble (FR); ROSTAING, Hervé, 38420 Le Versoud (FR)
(74) Mandataire: bioMérieux PI Groupement mandataires
(86) Numéro de dépôt international: PCT/FR2017/053121
(87) Numéro de publication internationale: WO 2018/091818

(56) Documents cités:
- EP-A1- 2 453 219
- WO-A1-2013/016211
- WO-A1-2015/054113
- WO-A2-2013/068760
- US-A1- 2009 129 978
- US-A1- 2012 156 716

## Description

La présente invention concerne un système et un procédé d'extraction pour extraire des micro-organismes contenus dans un échantillon, dans le but notamment d'identifier et/ou de caractériser les micro-organismes extraits par exemple par une analyse en spectrométrie de masse sur plaque d'analyse MALDI-TOF MS ou une analyse d'antibiogramme.

Un premier procédé connu pour extraire des micro-organismes contenus dans un échantillon positif, tel qu'une hémoculture positive, consiste à :
- déposer manuellement plusieurs gouttes de l'échantillon sur un ou plusieurs milieux de culture en boîtes de Pétri,
- incuber la ou les boîtes de Pétri pendant 18 à 48 heures selon le type de microorganismes,
- prélever manuellement une partie des colonies sur les boîtes de Pétri,
- déposer manuellement les colonies prélevées sur différents emplacements d'une plaque d'analyse MALDI-TOF MS, également appelée plaque d'analyse en spectrométrie de masse MALDI-TOF MS,
- effectuer une identification en spectrométrie de masse sur la plaque d'analyse MALDI-TOF MS.

Un deuxième procédé connu pour extraire des micro-organismes contenus dans un échantillon positif, tel qu'une hémoculture positive, consiste à :
- déposer manuellement plusieurs gouttes de l'échantillon sur un ou plusieurs milieux de culture en boîtes de Pétri,
- incuber la ou les boîtes de Pétri pendant 18 à 24 heures,
- prélever manuellement une partie des colonies sur les boîtes de Pétri,
- préparer une suspension concentrée et calibrée en densité optique de microorganismes à partir des colonies prélevées,
- effectuer une analyse d'antibiogramme sur la suspension prélevée.

Ces premier et deuxième procédés d'extraction connus sont longs et requièrent notamment la présence d'un opérateur expérimenté pour effectuer les nombreuses étapes manuelles.

Des automates ont ainsi été développés pour effectuer notamment les étapes de prélèvement et de dépôts mises en oeuvre pour les procédés décrits ci-dessus. Cependant, de tels automates requièrent un investissement pécuniaire très important pour un laboratoire d'analyse sans pour autant réduire de manière sensible la durée de ces procédés d'extraction.

Le document WO2013/016211 décrit un procédé pour extraire des micro-organismes contenus dans un échantillon positif, comprenant notamment l'utilisation d'un dispositif de filtration et de transfert pour isoler les micro-organismes contenus dans l'échantillon positif et pour les transférer notamment sur une plaque d'analyse MALDI-TOF MS.

Un tel procédé permet de réduire fortement la durée d'extraction de micro-organismes contenus dans un échantillon positif. Cependant, un tel procédé d'extraction requière encore la présence prolongée d'un opérateur expérimenté pour effectuer les différentes étapes manuelles durant toute la durée du procédé. Par ailleurs, le dépôt sur plaque d'analyse MALDI-TOF MS n'est pas standardisé en terme de dépôt, et ne permet pas aisément d'assurer la traçabilité des résultats.

La présente invention vise à remédier à ces inconvénients.

Le problème technique à la base de l'invention consiste donc à fournir un système d'extraction qui soit de structure simple et compact, tout en permettant une extraction rapide et automatisée de micro-organismes contenus dans un échantillon positif pour une analyse sur plaque d'analyse MALDI-TOF MS ou pour un antibiogramme à partir d'une suspension concentrée de microorganismes.

A cet effet, la présente invention concerne un système d'extraction pour extraire des micro-organismes contenus dans un échantillon, le système d'extraction comprenant :
- une barrette de support comprenant :
   - un puits d'échantillon destiné à recevoir un échantillon contenant des micro-organismes, tel qu'une hémoculture positive,
   - un puits de réactif destiné à contenir un tampon de lyse sélective,
   - au moins un puits de lavage destiné à contenir un réactif de lavage, et
   - au moins un logement de protection et de rétention,
- un élément de pipetage monté de manière amovible sur la barrette de support et s'étendant au moins en partie dans l'au moins un logement de protection et de rétention,
- un dispositif de filtration et de transfert destiné à capturer et accumuler des micro-organismes contenus dans l'échantillon et à transférer les micro-organismes capturés et accumulés, le dispositif de filtration et de transfert étant monté de manière amovible sur la barrette de support et s'étendant au moins en partie dans l'au moins un logement de protection et de rétention, le dispositif de filtration et de transfert comprenant :
   - un corps creux ayant une forme allongée et comportant une portion d'extrémité proximale et une portion d'extrémité distale, et
   - un élément de filtration fixé à la portion d'extrémité distale du corps creux.

Une telle configuration du système d'extraction lui assure une grande compacité, et permet en outre de le charger aisément, via un portoir, dans un automate apte notamment à manipuler l'élément de pipetage et le dispositif de filtration et de transfert en vue de réaliser automatiquement les différentes étapes d'extraction des micro-organismes contenus dans un échantillon positif introduit dans le puits d'échantillon.

En outre, l'utilisation du système d'extraction selon la présente invention ne requiert pas de formation ou de compétence particulière. En effet, les seules étapes manuelles devant être mises en oeuvre par un opérateur sont l'introduction de l'échantillon dans le puits d'échantillon, le positionnement de la barrette de support sur un portoir et le chargement du portoir dans l'automate. La simplicité d'usage du système d'extraction selon la présente invention permet en outre de traiter des hémocultures urgentes en service de nuit où le niveau de technicité est largement réduit.

De plus, associé à l'automate, le système d'extraction selon la présente invention permet de ne pas immobiliser un technicien durant l'ensemble des opérations manuelles d'extraction permettant de délivrer une plaque d'analyse MALDI-TOF MS avec des dépôts reproductibles et standardisés en terme de quantité déposée et directement utilisables dans le système d'analyse MALDI-TOF MS pour identifier ou caractériser les pathogènes présents dans l'échantillon.

Enfin, l'utilisation d'un dispositif de filtration et de transfert similaire à celui décrit dans le document WO2013/016211 assure une extraction rapide des micro-organismes contenus dans un échantillon chargé dans le puits d'échantillon, et un dépôt fiable des micro-organismes sur une plaque d'analyse MALDI-TOF MS.

En conséquence, le système d'extraction selon la présente invention permet une extraction rapide et automatisée des micro-organismes contenus dans un échantillon positif pour une analyse sur plaque d'analyse MALDI-TOF MS ou pour un antibiogramme.

Il convient en outre de noter que la configuration du système d'extraction permet de limiter grandement les coûts de fabrication de l'automate apte à réaliser automatiquement l'extraction des micro-organismes, du fait notamment que les différentes réactifs nécessaires sont déjà présents sur chaque barrette de support et n'ont donc pas à être stockés dans l'automate.

Le système d'extraction peut en outre présenter une ou plusieurs des caractéristiques suivantes, prises seules ou en combinaison.

Selon un mode de réalisation de l'invention, le puits d'échantillon, le puits de réactif et l'au moins un puits de lavage sont sensiblement alignés selon la direction d'extension de la barrette de support.

Selon un mode de réalisation de l'invention, la portion d'extrémité distale du corps creux et l'élément de filtration s'étendent dans l'au moins un logement de protection et de rétention.

Selon un mode de réalisation de l'invention, l'élément de pipetage comporte une portion d'extrémité distale s'étendant au moins en partie dans l'au moins un logement de protection et de rétention.

Selon un mode de réalisation de l'invention, l'au moins un logement de protection et de rétention est configuré pour retenir des gouttes de liquide, telles que des gouttes d'échantillon ou de réactifs, susceptibles de s'écouler par gravité depuis l'élément de pipetage et le dispositif de filtration et de transfert.

Selon un mode de réalisation de l'invention, l'élément de filtration est configuré pour retenir au moins une partie des micro-organismes contenus dans l'échantillon et pour laisser passer d'autres éléments contenus dans l'échantillon, tels que des éléments non micro-organiques, par exemple des débris cellulaires, ou les composés lysés du sang, tels que les hématies.

Selon un mode de réalisation de l'invention, l'élément de filtration est une membrane de filtration, telle qu'une membrane de type Pall SuporR (matériau PES), par exemple en forme de disque ou de dôme.

Selon un mode de réalisation de l'invention, l'élément de filtration est constitué d'un matériau sélectionné dans le groupe comprenant notamment le polyéthersulfone, les fibres de verre, l'acétate de cellulose et la cellulose régénérée.

Selon un mode de réalisation de l'invention, l'élément de filtration présente une taille de pores comprise entre 0,1 et 10 µm, préférentiellement de 0,2 à 0,8 µm et très préférentiellement de 0,45 µm.

Selon un mode de réalisation de l'invention, l'élément de filtration présente une épaisseur comprise entre 0,08 et 0,14 mm.

Selon un mode de réalisation de l'invention, l'élément de filtration a une forme cylindrique de diamètre adapté au diamètre d'un emplacement d'une plaque d'analyse MALDI-TOF MS, et présente une partie distale plate ou hémisphérique.

Selon un mode de réalisation de l'invention, l'élément de filtration est de forme convexe.

Selon un mode de réalisation de l'invention, le corps creux comporte une portion effilée convergeant vers la portion d'extrémité distale du corps creux.

Selon un mode de réalisation de l'invention, la portion d'extrémité distale du corps creux est cylindrique, évasée ou aplatie.

Selon un mode de réalisation de l'invention, la portion d'extrémité proximale du corps creux est configurée pour être reliée à une source de dépression.

Selon un mode de réalisation de l'invention, la portion d'extrémité proximale du corps creux comporte une partie de retenue. La partie de retenue est par exemple une collerette de retenue.

Selon un mode de réalisation de l'invention, l'élément de filtration s'étend au moins en partie à l'extérieur du corps creux. Par exemple, l'élément de filtration s'étend ou fait saillie à partir de la portion d'extrémité distale du corps creux.

Selon un mode de réalisation de l'invention, le dispositif de filtration et de transfert comporte un matériau absorbant disposé dans le corps creux.

Selon un mode de réalisation de l'invention, le matériau absorbant est au moins en partie en contact avec l'élément de filtration.

Selon un mode de réalisation de l'invention, le matériau absorbant est sélectionné dans le groupe comprenant le coton, la fibre de cellulose, le polyester, l'agglomérat de polyéthylène, une résine poreuse absorbante, un gel de silice, un hydrogel, un tamis moléculaire, une zéolite ou encore d'autres matériaux absorbants.

Selon un mode de réalisation de l'invention, le dispositif de filtration et de transfert comporte des billes de verre reposant sur le matériau absorbant.

Selon un mode particulier de réalisation de l'invention, le matériau absorbant est constitué d'un bloc monolithique fabriqué en agglomérat de billes plastique (par exemple type polyéthylène ou polyester) remplaçant avantageusement l'ensemble billes de verre et matériau absorbant à l'arrière de la membrane de filtration.

Selon un mode de réalisation de l'invention, le système d'extraction comprend en outre un puits de relargage, également appelé puits de récupération, monté, par exemple de manière amovible, sur la barrette de support ou inséré directement dans le portoir.

Selon un mode de réalisation de l'invention, le puits de relargage comporte une couche interne en silicone réticulé recouvrant au moins en partie la surface interne du puits de relargage, et contient une quantité prédéterminée de solution saline, par exemple à 0,45 %. Le puits de relargage peut par exemple contenir de 250 à 400 µL de solution saline, préférentiellement 320 µL de solution saline à 0,45%.

Selon un mode de réalisation de l'invention, l'au moins un puits de lavage contient un réactif de lavage et le puits de réactif contient un tampon de lyse sélective.

Selon un mode de réalisation de l'invention, le puits de réactif et l'au moins un puits de lavage sont chacun obturés par un élément d'obturation.

Selon un mode de réalisation de l'invention, chaque élément d'obturation est un film d'obturation, tel qu'un opercule d'obturation. Chaque élément d'obturation peut par exemple être en aluminium.

Selon un mode de réalisation de l'invention, le système d'extraction comprend en outre un organe d'obturation déplaçable ou déformable entre une position d'obturation dans laquelle l'organe d'obturation obture le puits d'échantillon et une position de libération dans laquelle l'organe d'obturation libère au moins en partie le puits d'échantillon. Ces dispositions permettent notamment de garantir la fermeture du puits échantillon lors du transport de la barrette de support ou de la manipulation d'un portoir équipé de la barrette de support. Elles permettent de garantir l'intégrité de l'échantillon ainsi que la sécurité du manipulateur lors de tout déplacement au cours des manipulations (par exemple de la hotte biologique à l'instrument).

Selon un mode de réalisation de l'invention, l'organe d'obturation est configuré pour être déplacé dans la position de libération lorsque la barrette de support est positionnée sur un portoir et que le portoir est chargé dans un automate. Ces dispositions assurent une ouverture complète du puits d'échantillon lors du chargement du portoir dans l'automate et une fermeture complète du puits d'échantillon lors du retrait du portoir de l'automate, et ce sans intervention de l'opérateur.

Selon un mode de réalisation de l'invention, la barrette de support est pourvue d'un code d'identification, tel qu'un code-barres. Ces dispositions assurent une traçabilité optimale des résultats d'analyse obtenus pour chaque patient.

Selon un mode de réalisation de l'invention, la barrette de support est en matière plastique.

Selon un mode de réalisation de l'invention, le corps creux du dispositif de filtration et de transfert est en matière plastique, et par exemple en polystyrène cristal injecté ou polypropylène injecté.

Selon un mode de réalisation de l'invention, le corps creux du dispositif de filtration et de transfert présente un diamètre interne compris entre 2 mm et 10 mm, et une longueur comprise entre 5 cm et 15 cm. La longueur totale du dispositif de filtration et de transfert est par exemple de 9 cm afin d'avoir un volume interne suffisant pour contenir l'ensemble des fluides aspirés durant le procédé et constituer ainsi un réservoir de rétention desdits fluides. La partie distale du dispositif de filtration et de transfert a avantageusement un diamètre effilé sur quelques cm de longueur, lui permettant de i) entrer profondément dans les différents tubes du système d'extraction, ii) s'adapter au diamètre des emplacements d'une plaque d'analyse MALDI-TOF MS pour l'opération de transfert.

Selon un mode de réalisation de l'invention, le corps creux du dispositif de filtration et de transfert est rigide ou semi-rigide.

Selon un mode de réalisation de l'invention, la barrette de support comprend une pluralité de puits de lavage destinés chacun à contenir un réactif de lavage. La barrette de support peut par exemple comprendre deux ou trois, voire plus, puits de lavage dont le volume et la composition des réactifs seront adaptés si besoin à l'application ou au type d'échantillon à extraire (urine, pus, liquide synovial, etc... cultivé préalablement en milieu de croissance liquide).

Selon un mode de réalisation de l'invention, la barrette de support comporte une partie de protection délimitant l'au moins un logement de protection et de rétention.

Selon un mode de réalisation de l'invention, la barrette de support comporte un logement de protection et de rétention dans lequel s'étendent au moins en partie l'élément de pipetage et le dispositif de filtration et de transfert.

Selon un mode de réalisation de l'invention, la barrette de support comporte un premier logement de protection et de rétention et un deuxième logement de protection et de rétention dans lesquels s'étendent au moins en partie respectivement l'élément de pipetage et le dispositif de filtration et de transfert.

La présente invention concerne en outre un procédé d'identification et/ou de caractérisation de micro-organismes contenus dans un échantillon, le procédé d'identification et/ou de caractérisation comprenant les étapes suivantes :
- prévoir un système d'extraction selon l'invention, dans lequel le puits de réactif contient un tampon de lyse sélective et l'au moins un puits de lavage contient un réactif de lavage,
- introduire une quantité prédéterminée d'un échantillon contenant des micro-organismes dans le puits d'échantillon,
- prélever une quantité prédéterminée du tampon de lyse sélective contenu dans le puits de réactif à l'aide de l'élément de pipetage,
- optionnellement vérifier la présence et le niveau de remplissage de l'échantillon dans le puits d'échantillon par une méthode optique,
- introduire dans le puits d'échantillon le tampon de lyse sélective prélevé à l'aide de l'élément de pipetage, et éventuellement mélanger l'échantillon et le tampon de lyse sélective dans ledit puits d'échantillon à l'aide de l'élément de pipetage,
- lyser des cellules et/ou des particules non micro-organiques contenues dans l'échantillon et/ou lyser des micro-organismes prédéterminés contenus dans l'échantillon de manière à fournir un échantillon lysé, également appelé lysat,
- immerger au moins en partie le dispositif de filtration et de transfert dans l'échantillon lysé contenu dans le puits d'échantillon,
- filtrer sous aspiration au moins une partie de l'échantillon lysé à travers l'élément de filtration,
- capturer et accumuler des micro-organismes dans ou sur l'élément de filtration,
- immerger au moins en partie le dispositif de filtration et de transfert dans le réactif de lavage contenu dans l'au moins un puits de lavage,
- laver, dans le réactif de lavage contenu dans l'au moins un puits de lavage, l'élément de filtration et les micro-organismes capturés et accumulés,
- transférer, à l'aide du dispositif de filtration et de transfert, les micro-organismes lavés sur un support d'analyse ou dans un récipient,
- analyser les micro-organismes transférés de manière à les identifier et/ou les caractériser.

Selon un mode de mise en oeuvre du procédé d'extraction, le volume d'échantillon introduit dans le puits d'échantillon peut varier entre 0,5 et 1,2 mL, et le volume de tampon de lyse sélective prélevé est alors modifié pour respecter un ratio 2:1 (par exemple si 1,2mL d'échantillon est introduit dans le puits d'échantillon, alors 600 µL de tampon de lyse sélective est prélevé).

Selon un mode de mise en oeuvre du procédé d'extraction, l'étape d'introduction de l'échantillon consiste à introduire 1 mL d'échantillon dans le puits d'échantillon, l'échantillon étant préférentiellement de l'hémoculture positive mais le procédé est applicable aux autres fluides corporelles stériles préalablement cultiver en milieux de croissance liquide afin d'augmenter la concentration des pathogènes potentiellement présents avant analyse.

Selon un mode de mise en oeuvre du procédé d'extraction, l'étape de prélèvement du tampon de lyse sélective consiste à prélever 500 µL de tampon de lyse sélective dans le puits de réactif. Ces 500µL sont mélangés et incubés avec le mL d'échantillon présent dans le puits d'échantillon, afin de lyser les éléments figurés sur sang notamment les hématies.

Selon un mode de mise en oeuvre du procédé d'extraction, l'étape de mélange de l'échantillon et du tampon de lyse sélective consiste à réaliser plusieurs cycles de mélange, chaque cycle de mélange consistant à aspirer et refouler une quantité prédéterminée du mélange contenu dans le puits d'échantillon, par exemple 500 µL (si le volume d'échantillon est de 1mL, respecter un ratio 1:2), à l'aide de l'élément de pipetage.

Selon un mode de mise en oeuvre du procédé d'extraction, l'étape de lyse comprend une étape consistant à incuber le mélange pendant une durée prédéterminée, par exemple entre 1 et 5 minutes, préférentiellement pendant 2 minutes, à température ambiante de manière à fournir l'échantillon débarrassé des éléments n'étant pas des micro-organismes. Par exemple, dans le cas d'un échantillon d'hémoculture, cette étape consiste à lyser les éléments figurés du sang, notamment les hématies.

Selon un mode de mise en oeuvre du procédé d'extraction, ce dernier comprend une étape préalable à l'étape d'immersion du dispositif de filtration et de transfert dans l'échantillon lysé et consistant à appliquer une dépression dans le corps creux du dispositif de filtration et de transfert.

Selon un mode de mise en oeuvre du procédé d'extraction, l'étape d'application d'une dépression dans le corps creux consiste à appliquer une dépression entre -50 mbar et -980 mbar par rapport à la pression atmosphérique, préférentiellement à environ -600 mbar par rapport à la pression atmosphérique.

Selon un mode de mise en oeuvre du procédé d'extraction, l'étape de filtration sous aspiration est réalisée par exemple pendant 2 minutes.

Selon un mode de mise en oeuvre du procédé d'extraction, le support d'analyse est une plaque d'analyse MALDI-TOF MS ou une lame de test.

Selon un mode de mise en oeuvre du procédé d'extraction, le récipient est formé par le puits de relargage prévu sur la barrette de support ou inséré directement dans le portoir.

Selon un mode de mise en oeuvre du procédé d'extraction, ce dernier comprend une étape consistant à maintenir la dépression dans le corps creux pendant les différentes étapes d'immersion du dispositif de filtration et de transfert, l'étape de filtration et l'étape de lavage.

Selon un mode de mise en oeuvre du procédé d'extraction, l'étape de lavage comprend l'étape suivante :
- déplacer pendant une durée prédéterminée, par exemple entre 30 secondes et 5 minutes, préférentiellement pendant 2 minutes, le dispositif de filtration et de transfert dans l'au moins un puits de lavage, par exemple selon un mouvement alternatif en Z.

Selon un mode de mise en oeuvre du procédé d'extraction, l'étape de déplacement du dispositif de filtration et de transfert dans l'au moins un puits de lavage consiste à réaliser plusieurs cycles de déplacement du dispositif de filtration et de transfert dans l'au moins un puits de lavage. Avantageusement, le dispositif de filtration et de transfert est extrait complètement hors de l'au moins un puits de lavage à chaque cycle de déplacement, par exemple à une fréquence d'environ 0,5 à 2 Hz, préférentiellement 1 Hz.

Selon un mode de mise en oeuvre du procédé d'extraction, ce dernier comprend une étape de collecte réalisée entre l'étape de lavage et l'étape de transfert et comprenant les étapes suivantes :
- retirer le dispositif de filtration et de transfert hors du réactif de lavage contenu dans l'au moins un puits de réactif, et
- déplacer la portion d'extrémité distale du corps creux transversalement à la direction d'extension du corps creux de telle sorte que la portion d'extrémité distale du corps creux entre en contact avec une paroi interne de l'au moins un puits de lavage.

Ces dispositions permettent de collecter, dans l'au moins un puits de lavage, des gouttes résiduelles de lavage présentes au niveau de la portion d'extrémité distale du dispositif de filtration et de transfert, et donc d'éviter le dépôt ultérieur d'une quantité trop importante de liquide sur le support d'analyse.

Selon un mode de mise en oeuvre du procédé d'extraction, l'étape de collecte comprend une étape consistant à maintenir la portion d'extrémité distale du corps creux contre la paroi interne de l'au moins un puits de lavage pendant une durée prédéterminée, par exemple pendant 5 à 20 secondes, préférentiellement 10 secondes. Avantageusement, la dépression dans le corps creux est maintenue pendant l'étape de maintien afin d'aspirer tout ou partie du liquide résiduel accroché à la partie distale du dispositif de filtration et de transfert.

Selon un mode de mise en oeuvre du procédé d'extraction, ce dernier comprend une étape préalable à l'étape de transfert, et avantageusement postérieure à l'étape de maintien de la portion d'extrémité distale du corps creux contre la paroi interne de l'au moins un puits de lavage, et consistant à mettre le corps creux à pression atmosphérique ou en légère surpression.

Selon un mode de mise en oeuvre du procédé d'extraction, l'étape d'immersion du dispositif de filtration et de transfert dans le réactif de lavage contenu dans l'au moins un puits de lavage consiste à plonger le dispositif de filtration et de transfert plus profondément, par exemple de 1 à 3 mm plus profond, que dans l'échantillon lysé contenu dans le puits d'échantillon.

Selon un mode de mise en oeuvre du procédé d'extraction, l'étape de transfert comprend une étape consistant à déposer, à l'aide du dispositif de filtration et de transfert, au moins une partie des micro-organismes lavés sur un ou plusieurs emplacements prédéterminés, également appelés spots, d'une plaque d'analyse MALDI-TOF MS.

Selon un mode de mise en oeuvre du procédé d'extraction, ce dernier comprend en outre les étapes suivantes réalisées avant l'étape d'analyse :
- sécher, de manière contrôlée, les différents emplacements de la plaque d'analyse MALDI-TOF MS sur lesquels des micro-organismes ont été déposés,
- optionnellement, réaliser une prise d'image des emplacements sur lesquels ont été déposés des microorganismes, par exemple selon deux illuminations distinctes,
- ajouter une quantité prédéterminée de matrice, par exemple de 0,5 à 2 µL, préférentiellement 1 µL, sur chaque emplacement de la plaque d'analyse MALDI-TOF MS où des micro-organismes ont été déposés,
- sécher, de manière contrôlée, les différents emplacements de la plaque d'analyse MALDI-TOF MS sur lesquels la matrice a été déposée,
- optionnellement, réaliser une prise d'image des emplacements sur lesquels a été déposée la matrice, par exemple selon deux illuminations distinctes.

Selon un mode de mise en oeuvre du procédé d'extraction, la matrice est une matrice DHB, à savoir un acide 2,5 Dihydroxybenzoique, une matrice HCCA, à savoir un acide α-cyano-4-hydroxycinnamique, ou une matrice SA, à savoir un acidesinapinique (3,5-dimethoxy-4-hydroxycinnamique).

Selon un mode de mise en oeuvre du procédé d'extraction, ce dernier comprend une étape réalisée préalablement à l'étape d'ajout de la matrice et consistant à ajouter une quantité prédéterminée d'acide formique, par exemple de 0,5 à 2 µL, préférentiellement 0,5 à 1 µL, sur chaque emplacement de la plaque d'analyse MALDI-TOF MS où des micro-organismes ont été déposés. Ces dispositions permettent notamment d'améliorer les résultats d'identification pour les Gram positifs et les levures.

Selon un mode de mise en oeuvre du procédé d'extraction, l'étape de dépôt des micro-organismes lavés sur un ou plusieurs emplacements prédéterminés de la plaque d'analyse MALDI-TOF MS comprend une étape consistant à appliquer l'élément de filtration contre le ou chaque emplacement prédéterminé de la plaque d'analyse MALDI-TOF MS avec une force d'application contrôlée comprise entre 100 grammes et 1 kg par dispositif de filtration, préférentiellement 600 grammes.

Selon un mode de mise en oeuvre du procédé d'extraction, l'étape de dépôt des micro-organismes lavés sur un ou plusieurs emplacements prédéterminés de la plaque d'analyse MALDI-TOF MS comprend une étape consistant à tamponner une ou plusieurs fois l'élément de filtration sur le ou chaque emplacement prédéterminé de la plaque d'analyse MALDI-TOF MS. Ces dispositions permettent un dépôt d'une quantité suffisante de micro-organismes sur chaque emplacement prédéterminé de la plaque d'analyse MALDI-TOF MS.

Selon un mode de mise en oeuvre du procédé d'extraction, une étape de prise d'images des emplacements sur la plaque d'analyse MALDI-TOF MS peut être optionnellement effectuée ; selon le besoin de contrôle, ces étapes de prise d'images peuvent être effectuées immédiatement après le transfert des microorganismes ou après dépôt des réactifs acide formique et matrice afin de vérifier le présence effective d'un dépôt. De manière préférentiellement, ces étapes de prise d'images ont lieu après chacune des étapes de séchage afin de vérifier la qualité et la quantité du dépôt des microorganismes, la bonne interaction entre l'acide formique et le dépôt de microorganismes (présence d'une auréole), la bonne répartition et cristallisation de la matrice avec les microorganismes déposés sur la plaque d'analyse MALDI-TOF MS.

Selon un mode de mise en oeuvre du procédé d'extraction, l'étape de tamponnage consiste à tamponner plusieurs fois, par exemple entre 3 et 7 fois, l'élément de filtration sur le ou chaque emplacement prédéterminé de la plaque d'analyse MALDI-TOF MS selon une fréquence de tamponnage comprise entre 0,5 et 10 Hz.

Selon un mode de mise en oeuvre du procédé d'extraction, l'étape précédente peut être combinée et conjointe avec une séquence de mise sous pression du corps creux du dispositif de filtration et de transfert afin de faciliter le transfert des microorganismes de la membrane filtrante à la surface de la plaque d'analyse MALDI-TOF MS.

Selon un mode de mise en oeuvre du procédé d'extraction, l'étape d'analyse comprend une étape consistant à effectuer une analyse en spectrométrie de masse de type MALDI-TOF des micro-organismes transférés, et par exemple des micro-organismes déposés sur une plaque d'analyse MALDI-TOF MS.

Selon un mode de mise en oeuvre du procédé d'extraction, l'étape d'analyse comprend une étape consistant à insérer le support d'analyse dans un spectromètre de masse.

Selon un mode de mise en oeuvre du procédé d'extraction, l'étape de lavage comprend une étape consistant à séparer, isoler ou purifier les micro-organismes capturés et accumulés dans l'élément de filtration, par exemple en facilitant l'évacuation des autres composants qui peuvent être présents dans l'échantillon lysé, tels que des débris cellulaires, des éléments non micro-organiques, et qui seraient susceptibles de nuire à l'analyse ultérieure des micro-organismes.

Selon un mode de mise en oeuvre du procédé d'extraction, ce dernier comprend une étape, en début de procédé, consistant à percer des films d'obturation de l'au moins un puits de lavage et du puits de réactif à l'aide de l'élément de pipetage. Ces dispositions permettent notamment de créer une ouverture suffisante pour que le dispositif de filtration et de transfert puisse pénétrer dans le ou les différents puits de lavage sans endommager l'élément de filtration.

Selon un mode de mise en oeuvre du procédé d'extraction, l'étape de perçage consiste à percer l'au moins un puits de lavage puis le puits de réactif par un mouvement vertical et éventuellement horizontal de l'élément de pipetage.

Selon un mode de mise en oeuvre du procédé d'extraction, ce dernier comprend une étape consistant à détecter un code d'identification prévu sur la barrette d'extraction afin de l'associer au code d'identification prévu sur le conteneur contenant initialement l'échantillon (par exemple un flacon d'hémoculture positif) et ainsi assurer une traçabilité optimale des résultats d'analyse.

Selon un mode de mise en oeuvre du procédé d'extraction, ce dernier comprend une étape réalisée après l'étape de dépôt des micro-organismes sur la plaque d'analyse MALDI-TOF MS et avant l'étape d'ajout de la matrice et consistant à incuber la plaque d'analyse MALDI-TOF MS entre 40 et 80°C, par exemple à 50°C, pendant une durée prédéterminée, par exemple entre 2 et 6 minutes, préférentiellement 4 minutes, et ce de manière à sécher ou évaporer le réactif de lavage résiduel éventuellement présent sur le ou chaque emplacement prédéterminé.

Selon un mode de mise en oeuvre du procédé d'extraction, ce dernier comprend une étape réalisée après l'étape d'ajout de l'acide formique et consistant à incuber la plaque d'analyse MALDI-TOF MS entre 40 et 80°C, par exemple à 45°C ou à 55°C, pendant une durée prédéterminée, par exemple entre 5 et 7 minutes, préférentiellement 6 minutes, et ce de manière à accélérer l'évaporation des gouttes d'acide formique, tout en assurant un temps de contact suffisant de l'acide formique avec les micro-organismes présent sur la plaque d'analyse MALDI-TOF MS pour garantir une lyse efficace des membranes des microorganismes.

Selon un mode de mise en oeuvre du procédé d'extraction, ce dernier comprend une étape réalisée après l'étape d'ajout de la matrice et consistant à incuber la plaque d'analyse MALDI-TOF MS entre 40 et 80°Cpendant une durée prédéterminée, par exemple entre 1 et 3 minutes, préférentiellement 2 minutes.

Selon un mode de mise en oeuvre du procédé d'extraction, l'étape de dépôt des micro-organismes sur le ou les emplacements prédéterminés de la plaque d'analyse MALDI-TOF MS comprend une étape consistant à faire vibrer le dispositif de filtration et de transfert, et plus particulièrement à faire vibrer le dispositif de filtration et de transfert au moins lors du contact de l'élément de filtration sur le ou chaque emplacement prédéterminé de la plaque d'analyse MALDI-TOF MS. Ces dispositions permettent d'améliorer le transfert des micro-organismes (quantité et reproductibilité) de l'élément de filtration à la plaque d'analyse MALDI-TOF MS.

Selon un mode de mise en oeuvre du procédé d'extraction, l'étape de mise en vibration du dispositif de filtration et de transfert consiste à appliquer une vibration comprise entre 0,1 et 200 Hz, par exemple entre 10 et 100 Hz, préférentiellement 50 Hz, pendant une durée prédéterminée, et par exemple entre 1 et 5 secondes, préférentiellement pendant 2 secondes.

Selon un mode de mise en oeuvre du procédé d'extraction, l'étape de transfert comprend une étape consistant à remettre en suspension les micro-organismes lavés dans une solution saline, par exemple à 0,45 %, contenue dans le puits de relargage.

Selon un mode de mise en oeuvre du procédé d'extraction, l'étape de remise en suspension comprend les étapes suivantes :
- immerger au moins en partie le dispositif de filtration et de transfert dans la solution saline contenue dans le puits de relargage,
- mettre en contact de manière répétée, par exemple par un mouvement alternatif vertical du puits de relargage et/ou du dispositif de filtration et de transfert, l'élément de filtration avec la couche interne en silicone réticulé recouvrant au moins en partie la surface interne du puits de relargage pendant une durée prédéterminée, et avantageusement à une fréquence prédéterminée.

Selon un mode de mise en oeuvre du procédé d'extraction, ce dernier comprend une étape consistant à réaliser une mesure de densité optique de la solution saline contenue dans le puits de relargage et contenant les micro-organismes lavés. La mesure de densité optique peut par exemple être réalisée dans le puits de relargage au dessus de la couche interne en silicone réticulé ou directement dans l'élément de pipetage.

Selon un mode de mise en oeuvre du procédé d'extraction, ce dernier comprend une étape consistant à ajuster, par exemple à 0,5 McF, la concentration de micro-organismes dans la solution saline contenue dans le puits de relargage par ajout de solution saline dans le puits de relargage. Cette étape peut être effectuée avantageusement de manière automatique en pilotant l'ajout de solution saline calculé à partir de la mesure de densité optique réalisée dans le puits de relargage.

Selon un mode de mise en oeuvre du procédé d'extraction, ce dernier comprend les étapes suivantes :
- prélever, dans le puits de relargage, une quantité prédéterminée de la solution saline contenant les microorganismes préalablement ajustée à une valeur de densité optique de 0,5 McF, par exemple entre 100 et 300 µL et avantageusement 145 ou 285 µL selon le type de microorganismes (Gram-positif ou Gram-négatif),
- introduire la solution saline prélevée dans un récipient d'analyse contenant une quantité prédéterminée de solution saline, par exemple à 0,45%, afin d'obtenir une concentration de micro-organismes prédéterminée, par exemple de 0,05 ou 0,025 McF en fonction du type de micro-organismes (par exemple gram-négatif, gram-positif ou levures), pour préparer le lancement d'une analyse antibiogramme directement à partir de l'hémoculture positive sans passer par l'étape de mise en culture sur milieu.

Selon un mode de mise en oeuvre du procédé d'extraction, le récipient d'analyse contient entre 2 et 4 mL, préférentiellement 3 mL de solution saline à 0,45%.

Selon un mode de mise en oeuvre du procédé d'extraction, le récipient d'analyse est monté, par exemple de manière amovible, sur la barrette de support. Avantageusement, le récipient d'analyse est disposé à proximité du puits de relargage.

Selon un mode de mise en oeuvre du procédé d'extraction, l'étape d'analyse des micro-organismes transférés consiste à effectuer une analyse d'antibiogramme. Avantageusement, l'étape d'analyse comprend une étape consistant à transférer une quantité prédéterminée du consommable, contenu dans le récipient d'analyse et contenant les micro-organismes transférés, dans un analyseur d'antibiogramme, et effectuer une analyse d'antibiogramme sur le consommable transféré.

Selon une variante de mise en oeuvre du procédé d'extraction, l'étape de remise en suspension comprend une étape consistant à frotter l'élément de filtration sur une grille en Nylon préalablement mouillée avec une solution saline à 0,45%.

Selon une autre variante de mise en oeuvre du procédé d'extraction, l'étape de remise en suspension comprend les étapes suivantes :
- introduire au moins en partie le dispositif de filtration et de transfert dans le puits de relargage,
- découper l'élément de filtration, par exemple à l'aide d'une pièce de découpe insérée dans le puits de relargage,
- libérer l'élément de filtration dans la solution saline contenue dans le puits de relargage,
- mettre en mouvement le puits de relargage pendant une durée prédéterminée, par exemple durant 30 secondes à 1 minute, de manière à libérer les microorganismes de l'élément de filtration et à les remettre en suspension dans la solution saline.

Selon un mode de mise en oeuvre du procédé d'extraction, l'étape de découpe comprend une étape consistant à mettre en rotation le puits de relargage par rapport au dispositif de filtration et de transfert, par exemple sur un demi-tour.

Selon un mode de mise en oeuvre du procédé d'extraction, ce dernier comprend en outre les étapes suivantes :
- prévoir un portoir,
- positionner la barrette de support sur le portoir.

Selon un mode de mise en oeuvre du procédé d'extraction, l'étape de positionnement de la barrette de support comprend une étape consistant à déplacement automatiquement l'organe d'obturation de la position d'obturation à la position de libération.

Selon un mode de mise en oeuvre du procédé d'extraction, ce dernier comprend une étape consistant à prévoir un automate comprenant une partie de manipulation configurée pour saisir/relâcher et déplacer l'élément de pipetage monté sur la barrette de support et pour saisir/relâcher et déplacer le dispositif de filtration et de transfert monté sur la barrette de support.

Selon un mode de mise en oeuvre du procédé d'extraction, la partie de manipulation de l'automate prévu est configurée pour déplacer respectivement l'élément de pipetage et le dispositif de filtration et de transfert selon des déplacements cartésiens.

Selon un mode de mise en oeuvre du procédé d'extraction, l'automate prévu comprend une zone de réception configurée pour recevoir un portoir équipé de la barrette de support.

De toute façon l'invention sera bien comprise à l'aide de la description qui suit en référence aux dessins schématiques annexés représentant, à titre d'exemples non limitatifs, deux formes d'exécution de ce système d'extraction.
Figure 1 est une vue de côté d'un système d'extraction selon un premier mode de réalisation de l'invention.
Figure 2 est une vue en coupe du système d'extraction de la figure 1.
Figure 3 est une vue partielle en coupe d'une portion d'extrémité distale d'un dispositif de filtration et de transfert du système d'extraction de la figure 1.
Figure 4 est une vue en perspective d'un portoir équipé d'une pluralité de systèmes d'extraction de la figure 1.
Figure 5 est une vue en perspective d'un automate équipé du portoir de la figure 4.
Figure 6 est une vue partielle en perspective d'un système d'extraction selon un deuxième mode de réalisation de l'invention.
Figure 7 est une vue en perspective d'un portoir équipé d'une pluralité de systèmes d'extraction de la figure 6 et en outre de récipients additionnels.
Figure 8 est une vue en coupe du portoir de la figure 7, des éléments d'obturation configurés pour obturer un puits de réactif et des puits de lavage d'un système d'extraction équipant le portoir ayant cependant été ajoutés.
Figures 9 et 10 sont des vues partielles en perspective d'un système d'extraction selon un troisième mode de réalisation de l'invention.
Figure 11 est une vue en perspective d'une plaque d'analyse MALDI-TOF MS.

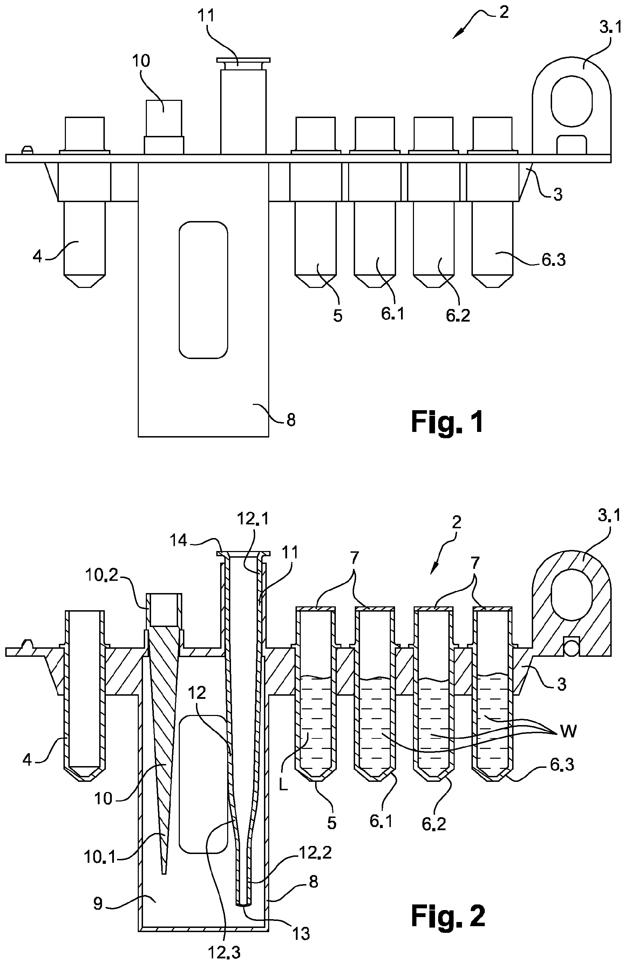

Les figures 1 à 3 représentent un système d'extraction 2, pour extraire des micro-organismes contenus dans un échantillon, selon un premier mode de réalisation de l'invention. L'échantillon peut par exemple être une culture microbiologique, telle qu'une hémoculture positive. L'échantillon peut en outre être un liquide corporel, tel qu'un liquide céphalo-rachidien, un liquide synovial, de l'urine ou encore du pus ayant préalablement été mis en culture en milieu de culture liquide adapté, afin d'augmenter la concentration de microorganismes par mL.

Le système d'extraction 2 comprend une barrette de support 3 réalisée par exemple en matière plastique, et avantageusement pourvue d'un code d'identification, tel qu'un code-barres.

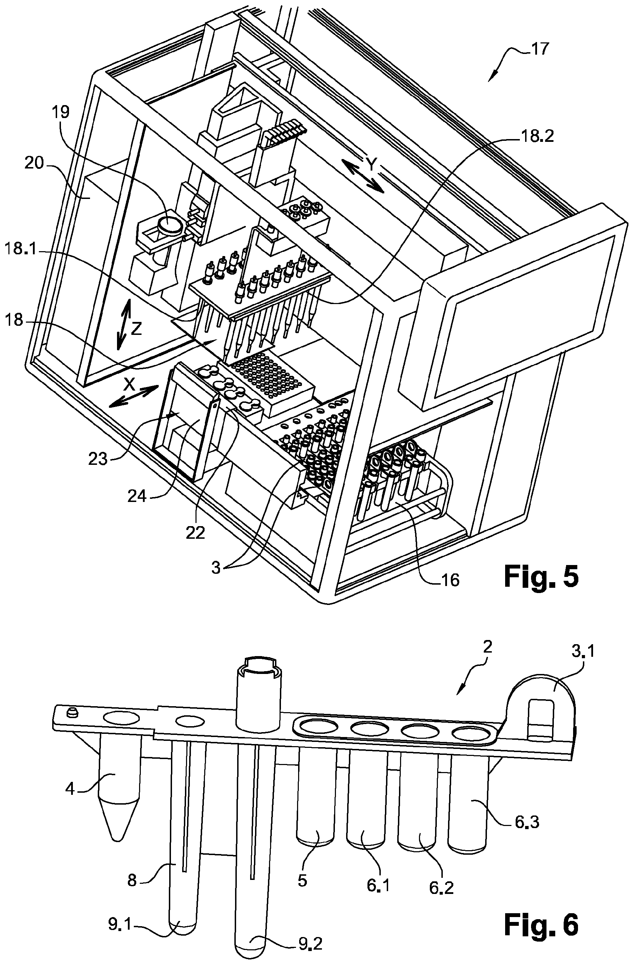

Comme montré plus particulièrement sur la figure 2, la barrette de support 3 comprend notamment un puits d'échantillon 4 destiné à recevoir un échantillon contenant des micro-organismes, un puits de réactif 5 contenant un tampon de lyse sélective L, et un ou plusieurs puits de lavage contenant chacun un réactif de lavage W. Selon le mode de réalisation représenté sur les figures 1 à 3, le puits d'échantillon 4, le puits de réactif 5 et le ou les puits de lavage sont rapportés sur la barrette de support 3. Cependant, selon un autre mode de réalisation du système d'extraction 2, la barrette de support 3, le puits d'échantillon 4, le puits de réactif 5 et le ou les puits de lavage pourraient être réalisés directement par moulage, et notamment par moulage par injection (voir la figure 6).

Selon le mode de réalisation représenté sur les figures 1 à 3, la barrette de support 3 comprend un puits de lavage 6.1 contenant un réactif de lavage ayant formulation identique aux formulations décrites dans le document WO2013/016211, un puits de lavage 6.2 contenant un réactif de lavage ayant formulation identique aux formulations décrites dans le document WO2013/016211, et un puits de lavage 6.3 contenant par exemple de l'eau déionisée stérile. Chacun des puits de lavage peut par exemple contenir 1400 µL du réactif de lavage respectif.

Selon un mode de réalisation de l'invention, le tampon de lyse sélective L contenu dans le puits de réactif 5 présente une formulation identique aux formulations décrites dans le document WO2013/016211.

Le puits d'échantillon 4, le puits de réactif 5 et le ou les puits de lavage 6.1, 6.2, 6.3 sont avantageusement alignés selon la direction d'extension de la barrette de support 3. De façon avantageuse, le puits de réactif 5 et le ou les puits de lavage 6.1, 6.2, 6.3 sont obturés par des opercules d'obturation 7 respectifs, constitués par exemple d'un complexe aluminium/polyéthylène thermosoudé à la surface des puits de réactif et de lavage.

La barrette de support 3 comprend également une partie de préhension 3.1 facilitant sa manipulation par un opérateur.

La barrette de support 3 comprend en outre une partie de protection 8 délimitant un logement de protection et de rétention 9 qui est ouvert vers le haut et dont la fonction sera précisée ci-après. Selon le mode de réalisation représenté sur les figures 1 à 3, la partie de protection 8 est disposée entre le puits d'échantillon 4 et le puits de réactif 5. Cette position est prévue afin de limiter au minimum les risques de souillures, lors du déplacement de l'élément de pipetage au-dessus de la barrette de support 3.

Le système d'extraction 2 comprend en outre un élément de pipetage 10, par exemple en matière plastique, monté de manière amovible sur la barrette de support 3, et s'étendant au moins en partie dans le logement de protection et de rétention 9. Selon le mode de réalisation représenté sur les figures 1 à 3, l'élément de pipetage 10 comporte une portion d'extrémité distale effilée 10.1 s'étendant dans le logement de protection et de rétention 9, et une portion d'extrémité proximale 10.2 configurée pour être reliée à une source de dépression ou un organe de déplacement de volume, tel qu'une seringue pilotée électroniquement. De façon avantageuse, le logement de protection et de rétention 9 est configuré pour retenir des gouttes de liquide, telles que des gouttes d'échantillon ou de réactifs, susceptibles de s'écouler par gravité depuis la portion d'extrémité distale effilée 10.1 de l'élément de pipetage 10. Une fois les opérations de pipetage effectuées, l'élément de pipetage 10 est remis en position dans son logement dans la barrette d'extraction 3.

Le système d'extraction 2 comprend également un dispositif de filtration et de transfert 11 destiné à capturer et accumuler des micro-organismes contenus dans l'échantillon et à transférer les micro-organismes capturés et accumulés notamment vers un support d'analyse ou récipient. Le dispositif de filtration et de transfert 11 est monté de manière amovible sur la barrette de support 3, et s'étend au moins en partie dans le logement de protection et de rétention 9.

Le dispositif de filtration et de transfert 11 comprend plus particulièrement un corps creux 12 ayant une forme allongée et comportant une portion d'extrémité proximale 12.1 et une portion d'extrémité distale 12.2, et un élément de filtration 13 fixé à la portion d'extrémité distale 12.2 du corps creux 12, par exemple par soudure et notamment par thermo-soudure ou par ultrasons, ou encore par solvant. La portion d'extrémité distale 12.2 du corps creux 12 et l'élément de filtration 13 s'étendent dans le logement de protection et de rétention 9, et le logement de protection et de rétention 9 est configuré pour retenir des gouttes d'échantillon ou de réactifs susceptibles de s'écouler par gravité depuis le dispositif de filtration et de transfert 11. Cependant, selon un autre mode de réalisation du système d'extraction 2 (voir les figures 6 à 8), la partie de protection 8 pourrait comporter deux logements de protection et de rétention 9.1, 9.2 dans lesquels seraient respectivement montées la portion d'extrémité distale 10.1 de l'élément de pipetage 10 et la portion d'extrémité distale 12.2 du corps creux 12 du dispositif de filtration et de transfert 11.

Le corps creux 12 du dispositif de filtration et de transfert 11 est avantageusement rigide ou semi-rigide, et peut être en matière plastique, et par exemple en polystyrène cristal injecté ou en polypropylène injecté.

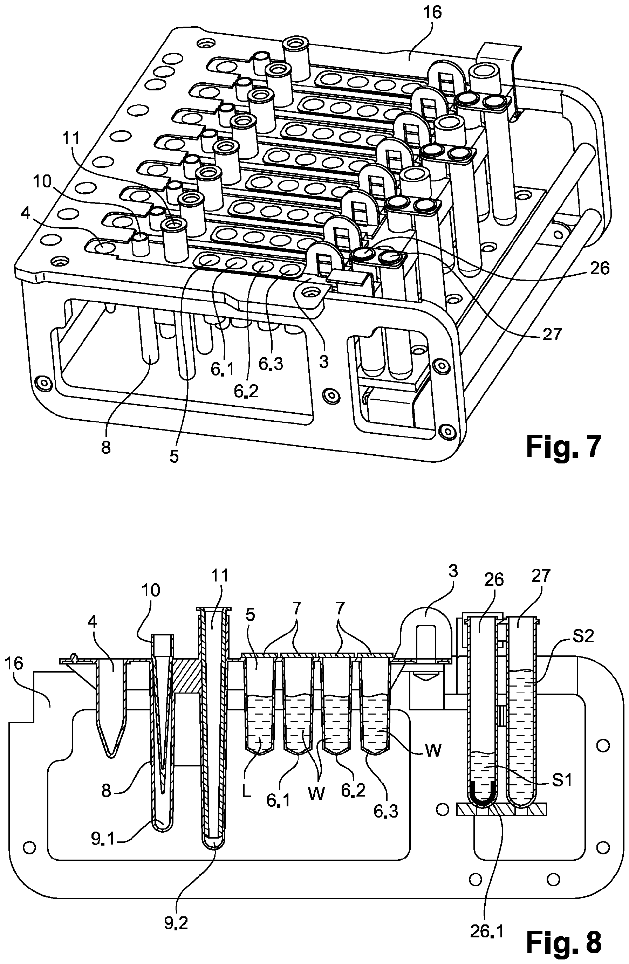

Selon le mode de réalisation représenté sur les figures 1 à 3, le corps creux 12 comporte une portion intermédiaire effilée 12.3 convergeant vers la portion d'extrémité distale 12.2 du corps creux 12, et la portion d'extrémité distale 12.2 est cylindrique. Cependant, le corps creux 12 pourrait avoir une forme différente, et la portion d'extrémité distale 12.2 pourrait notamment être effilée. Par exemple, selon le mode de réalisation représenté sur la figure 8, le corps creux 12 présente une section transversale qui diminue progressivement de la portion d'extrémité proximale 12.1 à la portion d'extrémité distale 12.2.

Il doit être noté que la portion d'extrémité proximale 12.1 du corps creux 12 est configurée pour être reliée à la source de dépression, et peut par exemple être pourvue d'une collerette de retenue 14. Cette collerette de retenue 14 sert également de plan de référence afin de précisément positionner dans un référentiel cartésien le dispositif de filtration et de transfert lors des étapes de transfert sur la plaque d'analyse MALDI-TOF MS.

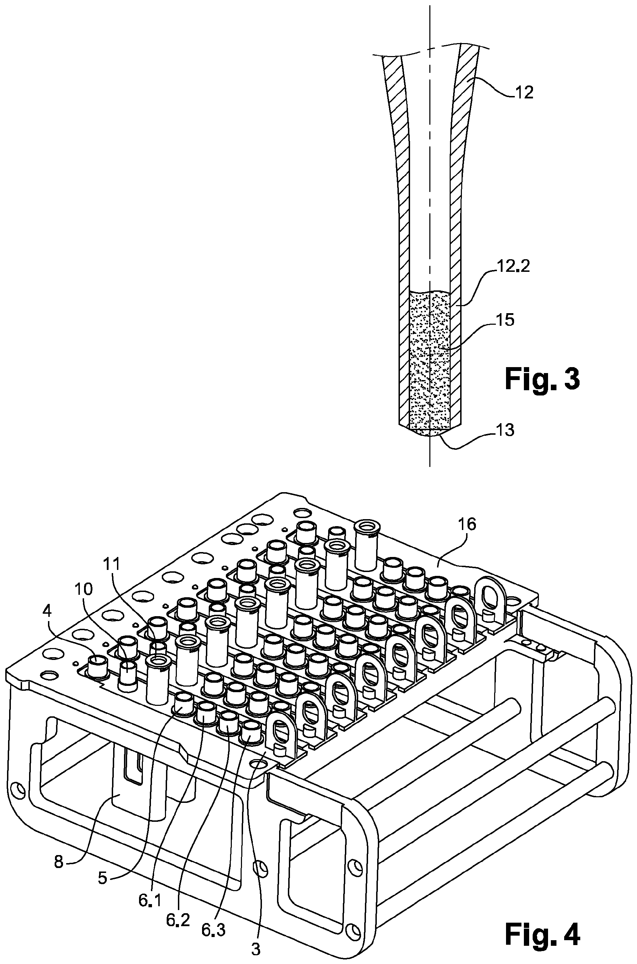

Comme montré plus particulièrement sur la figure 3, l'élément de filtration 13 s'étend au moins en partie à l'extérieur du corps creux 12. Par exemple, l'élément de filtration 13 fait saillie à partir de la portion d'extrémité distale 12.2 du corps creux 12. Selon le mode de réalisation représenté sur les figures 1 à 3, l'élément de filtration 13 est de forme convexe, et présente plus particulièrement une forme de dôme. Cependant, l'élément de filtration 13 pourrait présenter une autre forme, et par exemple être plat et présenter une forme de disque. L'élément de filtration 13 pourrait également être de forme cylindrique et présenter une partie distale plate ou hémisphérique. L'élément de filtration 13 pourrait de plus présenter un diamètre adapté au diamètre d'un emplacement d'une plaque d'analyse MALDI-TOF MS. L'élément de filtration 13 pourrait en outre être fabriqué in situ dans le corps creux 12.

L'élément de filtration 13 présente avantageusement une taille de pores comprise entre 0,1 et 10 µm, et par exemple d'environ 0,45 µm, et est plus particulièrement configuré pour retenir au moins une partie des micro-organismes contenus dans l'échantillon et pour laisser passer d'autres éléments contenus dans l'échantillon, tels que des éléments non micro-organiques, par exemple des débris cellulaires et les éléments figurés du sang préalablement lysés avec le réactif de lyse.

Le dispositif de filtration et de transfert 11 peut en outre comprendre un matériau absorbant 15 disposé dans le corps creux 12 et étant au moins en partie en contact avec l'élément de filtration 13. Le matériau absorbant 15 peut par exemple être sélectionné dans le groupe comprenant le coton, la fibre de cellulose, l'agrégat de billes de polyéthylène, le polyester, une résine absorbante, un gel de silice, un hydrogel, un tamis moléculaire, une zéolite ou encore d'autres matériaux absorbants.

Un premier procédé d'identification et/ou de caractérisation de micro-organismes contenus dans un échantillon à l'aide d'un système d'extraction 2 selon le premier mode de réalisation de l'invention va maintenant être décrit.

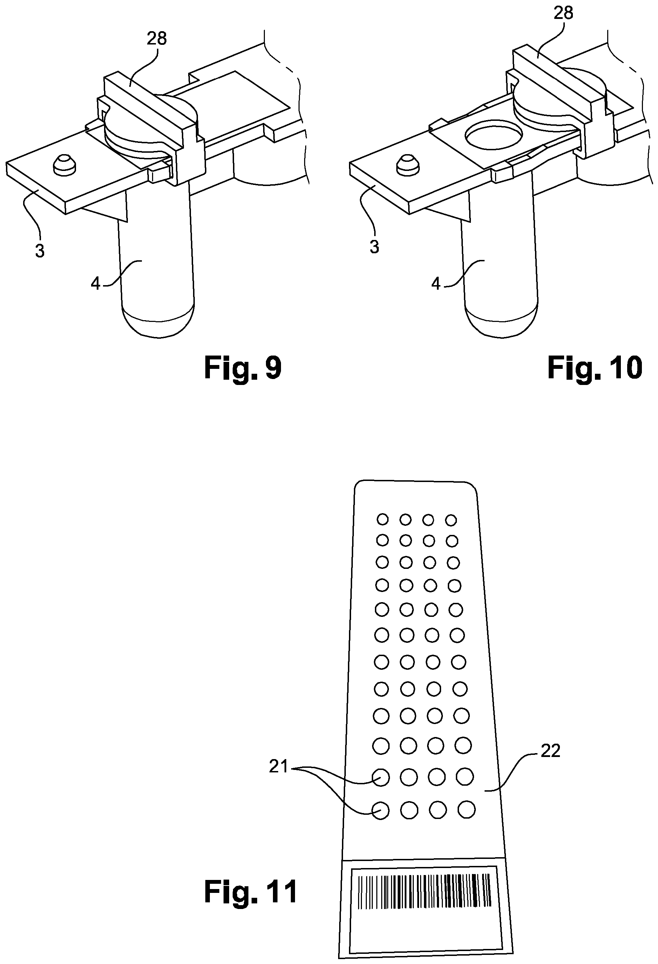

Le premier procédé d'identification et/ou de caractérisation comprend les étapes suivantes :
- détecter le code d'identification dont est pourvu le conteneur contenant initialement l'échantillon (par exemple un flacon d'hémoculture positif),
- charger la barrette de support 3 sur un portoir 16 (voir la figure 4),
- détecter le code d'identification dont est pourvue la barrette d'extraction 3,
- introduire une quantité prédéterminée, par exemple 1 mL, d'un échantillon contenant des micro-organismes, par exemple une hémoculture positive, dans le puits d'échantillon 4 du système d'extraction 2,
- charger le portoir 16 équipé de la barrette de support 3 dans un automate 17 (voir la figure 5) comprenant une partie de manipulation 18 configurée pour saisir/relâcher et déplacer l'élément de pipetage 10 monté sur la barrette de support 3 selon des déplacements cartésiens et pour saisir/relâcher et déplacer le dispositif de filtration et de transfert 11 monté sur la barrette de support 3 selon des déplacements cartésiens, la partie de manipulation 18 étant par exemple configurée pour déplacer l'élément de pipetage 10 et le dispositif de filtration et de transfert 11 de la ou de chaque barrette de support 3 chargée sur le portoir 16, selon l'axe Y et selon l'axe Z d'un repère cartésien, et comprenant par exemple plusieurs premiers éléments de manipulation 18.1 configurés chacun pour saisir/relâcher un élément de pipetage 10 respectif et plusieurs deuxièmes éléments de manipulation 18.2 configurés chacun pour saisir/relâcher un dispositif de filtration et de transfert 11 respectif, les premiers éléments de manipulation 18.1 étant par exemple alignés selon une première direction d'alignement et les deuxièmes éléments de manipulation 18.2 étant par exemple alignés selon une deuxième direction d'alignement sensiblement parallèle à la première direction d'alignement,
- optionnellement, réaliser, à l'aide d'une caméra 19 appartenant à l'automate 17, des prises d'images d'emplacements 21 prédéterminés d'une plaque d'analyse en spectrométrie de masse MALDI-TOF MS 22 disposée sur un module de support 23 de l'automate 17, avant la réalisation de dépôts sur ces emplacements 21 prédéterminés, le module de support 23 étant par exemple déplaçable selon l'axe X d'un repère cartésien,
- saisir l'élément de pipetage 10 monté sur la barrette de support 3 à l'aide de la partie de manipulation 18,
- percer les films d'obturation 7 des puits de lavage 6.1, 6.2, 6.3 et du puits de réactif 5 en progressant depuis le puits de lavage 6.3 vers le puits de réactif, par exemple à l'aide de l'élément de pipetage 10,
- prélever une quantité prédéterminée, par exemple 500 µL, du tampon de lyse sélective L contenu dans le puits de réactif 5 à l'aide de l'élément de pipetage 10,
- introduire, à l'aide de l'élément de pipetage 10 et dans le puits d'échantillon 4, le tampon de lyse sélective prélevé,
- mélanger l'échantillon et le tampon de lyse sélective contenus dans le puits d'échantillon 4 à l'aide de l'élément de pipetage 10,
- repositionner l'élément de pipetage 10 sur la barrette de support 3,
- incuber le mélange pendant une durée prédéterminée, par exemple entre 1 et 5 minutes, préférentiellement pendant 2 minutes, à température ambiante de manière à lyser des cellules et/ou des particules non micro-organiques contenues dans l'échantillon et/ou lyser des micro-organismes prédéterminés contenus dans l'échantillon afin de fournir un échantillon lysé, également appelé lysat,
- saisir le dispositif de filtration et de transfert 11 monté sur la barrette de support 3 à l'aide de la partie de manipulation 18,
- appliquer et maintenir une dépression entre -50 mbar et -980 mbar par rapport à la pression atmosphérique, préférentiellement à environ -600 mbar par rapport à la pression atmosphérique, dans le corps creux 12 du dispositif de filtration et de transfert 11, la dépression étant générée par une pompe 20 appartenant à l'automate 17 et reliée fluidiquement à la partie de manipulation 18, la pompe 20 étant par exemple une pompe à vide et à surpression, et pouvant par exemple être reliée à un ballast de vide et un ballast de pression,
- immerger au moins en partie la portion d'extrémité distale 12.2 du dispositif de filtration et de transfert 11 dans l'échantillon lysé contenu dans le puits d'échantillon 4,
- filtrer sous aspiration, par exemple pendant 2 minutes, au moins une partie de l'échantillon lysé à travers l'élément de filtration 13,
- capturer et accumuler des micro-organismes dans l'élément de filtration 13,
- immerger au moins en partie la portion d'extrémité distale 12.2 du dispositif de filtration et de transfert 11 dans le réactif de lavage contenu dans le puits de lavage 6.1,
- laver, par exemple par un cycle de mouvement alternatif vertical durant 30 s à 2 mn, préférentiellement 1 mn, dans le réactif de lavage contenu dans le puits de lavage 6.1, l'élément de filtration 13 et les micro-organismes capturés et accumulés,
- immerger au moins en partie la portion d'extrémité distale 12.2 du dispositif de filtration et de transfert 11 dans le réactif de lavage contenu dans le puits de lavage 6.2,
- laver, par exemple par un cycle de mouvement alternatif vertical durant 30 s à 2 mn, puis de manière statique durant 30 s à 5 mn, préférentiellement 3 mn, dans le réactif de lavage contenu dans le puits de lavage 6.2, l'élément de filtration 13 et les micro-organismes capturés et accumulés,
- immerger au moins en partie la portion d'extrémité distale 12.2 du dispositif de filtration et de transfert 11 dans le réactif de lavage contenu dans le puits de lavage 6.3,
- laver, par exemple par un cycle de mouvement alternatif vertical durant 30 s à 2 mn, puis de manière statique durant 30 s à 5 mn, préférentiellement 3 mn, dans le réactif de lavage contenu dans le puits de lavage 6.3, l'élément de filtration 13 et les micro-organismes capturés et accumulés,
- retirer la partie distale du dispositif de filtration et de transfert 11 hors du réactif de lavage contenu dans le puits de lavage 6.3 et déplacer la portion d'extrémité distale 12.2 du corps creux 12 transversalement à la direction d'extension du corps creux 12 de telle sorte que la portion d'extrémité distale du corps creux 12 entre en contact avec la paroi interne du puits de lavage 6.3 et que les gouttes résiduelles de lavage présentes au niveau de la portion d'extrémité distale 12.2 du corps creux 12 soient collectées dans le puits de lavage 6.3,
- maintenir la portion d'extrémité distale 12.2 du corps creux 12 contre la paroi interne du puits de lavage 6.3 pendant une durée prédéterminée, par exemple pendant 5 à 15 secondes, en maintenant le niveau de dépression,
- remettre le corps creux 12 du dispositif de filtration et de transfert 11 à pression atmosphérique ou en légère surpression (50 à 200 mbars) le temps nécessaire à l'étape suivante,
- déposer, à l'aide du dispositif de filtration et de transfert 11, au moins une partie des micro-organismes lavés sur un ou plusieurs emplacements prédéterminés 21, également appelés spots, de la plaque d'analyse MALDI-TOF MS 22 (voir la figure 11),
- repositionner le dispositif de filtration et de transfert 11 sur la barrette de support 3,
- incuber la plaque d'analyse MALDI-TOF MS 22 entre 40 et 80°C, par exemple à 45°C ou à 55°C, pendant une durée prédéterminée, par exemple entre 2 et 6 minutes, préférentiellement 4 minutes, de manière à sécher ou évaporer le réactif de lavage résiduel éventuellement présent sur le ou chaque emplacement prédéterminé 21 de la plaque d'analyse MALDI-TOF MS 22, le module de support 23 pouvant à cet effet être pourvu d'au moins un élément chauffant 24,
- optionnellement, réaliser une prise d'image des emplacements prédéterminés 21 à l'aide de la caméra 19,
- optionnellement, ajouter une quantité prédéterminée d'acide formique, par exemple de 0,5 à 1 µL, préférentiellement 0,5 µL, sur chaque emplacement 21 de la plaque d'analyse MALDI-TOF MS 22 où des micro-organismes ont été déposés, par exemple à l'aide d'un dispositif de pipetage (non représenté sur les figures) appartenant à l'automate 17 et déplaçable par exemple selon l'axe Y et selon l'axe Z d'un repère cartésien,
- incuber la plaque d'analyse MALDI-TOF MS entre 40 et 80°C, par exemple à 45°C ou à 55°C, pendant une durée prédéterminée, par exemple entre 5 et 7 minutes, préférentiellement 6 minutes de manière à sécher chaque emplacement prédéterminé 21 de la plaque d'analyse MALDI-TOF MS 22,
- optionnellement, réaliser une prise d'image des emplacements 21 à l'aide de la caméra 19,
- ajouter une quantité prédéterminée de matrice, par exemple de 0,5 à 2 µL, préférentiellement 1 µL de matrice HCCA, sur chaque emplacement 21 de la plaque d'analyse MALDI-TOF MS 22 où des micro-organismes ont été déposés, par exemple à l'aide du dispositif de pipetage,
- incuber la plaque d'analyse MALDI-TOF MS 22 entre 40 et 80°C pendant une durée prédéterminée, par exemple entre 1 et 3 minutes, préférentiellement 2 minutes,
- optionnellement, réaliser une prise d'image des emplacements 21 à l'aide de la caméra 19,
- insérer la plaque d'analyse MALDI-TOF MS 22 dans un spectromètre de masse, et
- effectuer une analyse en spectrométrie de masse des micro-organismes transférés sur chaque emplacement prédéterminé 21 de la plaque d'analyse MALDI-TOF MS 22 de manière à identifier et/ou caractériser les micro-organismes.

Selon un mode de mise en oeuvre du premier procédé d'extraction, chaque étape de lavage comprend une étape consistant à déplacer pendant une durée prédéterminée, par exemple entre 30 secondes et 5 minutes, préférentiellement pendant 2 minutes, le dispositif de filtration et de transfert 11 dans le puits de lavage respectif, par exemple selon un mouvement alternatif en Z. Avantageusement, chaque étape de déplacement du dispositif de filtration et de transfert 11 consiste à réaliser plusieurs cycles de déplacement verticaux du dispositif de filtration et de transfert 11 dans le puits de lavage respectif. De façon avantageuse, le dispositif de filtration et de transfert 11 est extrait complètement hors du puits de lavage respectif à chaque cycle de déplacement, par exemple à une fréquence d'environ 0,5 à 2 Hz, et de préférence 1 Hz.

Selon un mode de mise en oeuvre du premier procédé d'extraction, l'étape d'immersion du dispositif de filtration et de transfert 11 dans le réactif de lavage contenu dans le puits de lavage 6.1 consiste à plonger le dispositif de filtration et de transfert 11 plus profondément, par exemple de 1 à 3 mm plus profond, que dans l'échantillon lysé contenu dans le puits d'échantillon 4, l'étape d'immersion du dispositif de filtration et de transfert 11 dans le réactif de lavage contenu dans le puits de lavage 6.2 consiste à plonger le dispositif de filtration et de transfert 11 plus profondément, par exemple de 1 à 3 mm plus profond, que dans le réactif de lavage contenu dans le puits de lavage 6.1, et l'étape d'immersion du dispositif de filtration et de transfert 11 dans le réactif de lavage contenu dans le puits de lavage 6.3 consiste à plonger le dispositif de filtration et de transfert 11 plus profondément, par exemple de 1 à 3 mm plus profond, que dans le réactif de lavage contenu dans le puits de lavage 6.2.

Selon un mode de mise en oeuvre du premier procédé d'extraction, l'étape de dépôt des micro-organismes lavés sur un ou plusieurs emplacements prédéterminés 21 de la plaque d'analyse MALDI-TOF MS 22 comprend une étape consistant à tamponner plusieurs fois, par exemple entre 3 et 7 fois, l'élément de filtration 13 sur le ou chaque emplacement prédéterminé 21 de la plaque d'analyse MALDI-TOF MS 22 avec une force d'application comprise entre 100 grammes et 1kg, préférentiellement 600 grammes, et selon une fréquence de tamponnage comprise entre 0,5 et 10 Hz. Ces dispositions permettent un dépôt d'une quantité suffisante de micro-organismes sur chaque emplacement prédéterminé de la plaque d'analyse MALDI-TOF MS 22.

Selon un mode de mise en oeuvre du premier procédé d'extraction, l'étape de dépôt des micro-organismes sur le ou les emplacements prédéterminés 21 de la plaque d'analyse MALDI-TOF MS 22 comprend une étape consistant à faire vibrer le dispositif de filtration et de transfert 11 au moins lors du contact de l'élément de filtration 13 sur le ou chaque emplacement prédéterminé de la plaque d'analyse MALDI-TOF MS. Ces dispositions permettent d'améliorer le transfert des micro-organismes (quantité et reproductibilité) de l'élément de filtration 13 à la plaque d'analyse MALDI-TOF MS, notamment pour les microorganismes difficiles à détacher et identifier ou connus pour générer de faibles concentrations lors de leur croissance en flacon d'hémoculture.

Selon un mode de mise en oeuvre du premier procédé d'extraction, l'étape de mise en vibration du dispositif de filtration et de transfert 11 consiste à appliquer au dispositif de filtration et de transfert 11 une vibration comprise entre 0,1 et 200 Hz, par exemple entre 10 et 100 Hz, préférentiellement 50 Hz, pendant une durée prédéterminée, et par exemple pendant 2 secondes.

Selon un mode de mise en oeuvre du premier procédé d'extraction, l'étape de dépôt des micro-organismes sur le ou les emplacements prédéterminés 21 de la plaque d'analyse MALDI-TOF MS 22 comprend une étape consistant à appliquer un cycle de surpression (ajustement de la pression, du nombre d'impulsion, du rapport cyclique ou de la durée du cycle d'impulsion de pression), par rapport à la pression atmosphérique, dans le corps creux 12 du dispositif de filtration et de transfert 11 lors du contact de l'élément de filtration 13 sur le ou chaque emplacement prédéterminé 21 de la plaque d'analyse MALDI-TOF MS 22 ou entre deux contacts de l'élément de filtration 13 sur la plaque d'analyse MALDI-TOF MS 22. Ces dispositions permettent d'améliorer le transfert des micro-organismes de l'élément de filtration 13 à la plaque d'analyse MALDI-TOF MS 22.

Selon un mode de mise en oeuvre du premier procédé d'extraction, l'étape de mélange de l'échantillon et du tampon de lyse sélective consiste à réaliser plusieurs cycles de mélange, chaque cycle de mélange consistant à aspirer et refouler une quantité prédéterminée du mélange contenu dans le puits d'échantillon 4, par exemple 500 µL, à l'aide de l'élément de pipetage.

Selon un mode de mise en oeuvre du premier procédé d'extraction, ce dernier comprend une étape réalisée préalablement à l'étape d'ajout de la matrice et consistant à ajouter une quantité prédéterminée d'acide formique, par exemple de 0,5 à 2 µL, préférentiellement 0,5 à 1 µL, sur chaque emplacement de la plaque d'analyse MALDI-TOF MS où des micro-organismes ont été déposés. Ces dispositions permettent notamment d'améliorer les résultats d'identification pour les bactéries Gram positives et les levures.

Selon un mode de mise en oeuvre du premier procédé d'extraction, ce dernier comprend une étape réalisée après l'étape d'ajout de l'acide formique et consistant à incuber la plaque d'analyse MALDI-TOF MS 22 entre 40 et 80°C, par exemple à 45°C ou à 55°C, pendant une durée prédéterminée, par exemple entre 5 et 7 minutes, préférentiellement 6 minutes, et ce de manière à accélérer l'évaporation des gouttes d'acide formique, tout en assurant un contact suffisant de l'acide formique avec les micro-organismes présent sur la plaque d'analyse MALDI-TOF MS pour garantir une lyse efficace.

Selon un mode de mise en oeuvre du premier procédé d'extraction, ce dernier comprend une étape de prise d'image, par exemple selon deux types d'illumination distincts, des emplacements prédéterminés à différentes étapes du procédé et préférentiellement après chaque étape de séchage (à vide, microorganismes, acide formique, matrice) afin de contrôler le procédé à chaque étape (notamment les point suivants : transfert correct des microorganismes, localisation et mélange correct de l'acide formique et de la matrice avec les microorganismes déposés, niveau de cristallisation satisfaisant de la matrice avec les microorganismes).

Selon un mode de mise en oeuvre du premier procédé d'extraction, ce dernier comprend une étape de prise d'image du portoir après insertion dans l'automate à l'aide d'une caméra bas coût (webcam) incorporée dans l'automate. Cette prise d'image permet, par un traitement d'image adéquate, de vérifier la position du portoir, le nombre et la bonne insertion des barrettes, la bonne présence des éléments de pipetage et de filtration avant le lancement d'un cycle d'extraction.

Un deuxième procédé d'identification et/ou de caractérisation de micro-organismes contenus dans un échantillon à l'aide d'un système d'extraction 2 selon le premier mode de réalisation de l'invention va maintenant être décrit.

Le deuxième procédé d'identification et/ou de caractérisation comprend les étapes suivantes :
- charger la barrette de support 3 et un puits de relargage 26 sur un portoir 16,
- détecter le code d'identification dont est pourvue la barrette d'extraction 3 et détecter le code d'identification dont est pourvu le conteneur contenant l'échantillon (par exemple le flacon d'hémoculture),
- introduire une quantité prédéterminée, par exemple 1 mL, d'un échantillon contenant des micro-organismes, par exemple une hémoculture positive, dans le puits d'échantillon 4 du système d'extraction 2,
- charger le portoir 16 équipé de la barrette de support 3 dans un automate,
- saisir l'élément de pipetage 10 monté sur la barrette de support 3 à l'aide de la partie de manipulation 18,
- prélever une quantité prédéterminée, par exemple 500 µL, du tampon de lyse sélective L contenu dans le puits de réactif 5 à l'aide de l'élément de pipetage 10,
- introduire, à l'aide de l'élément de pipetage 10 et dans le puits d'échantillon 4, le tampon de lyse sélective prélevé,
- mélanger l'échantillon et le tampon de lyse sélective contenus dans le puits d'échantillon 4 à l'aide de l'élément de pipetage 10,
- repositionner l'élément de pipetage 10 sur la barrette de support 3,
- incuber le mélange pendant une durée prédéterminée, par exemple entre 1 et 5 minutes, préférentiellement pendant 2 minutes, à température ambiante de manière à lyser des cellules et/ou des particules non micro-organiques contenues dans l'échantillon et/ou lyser des micro-organismes prédéterminés contenus dans l'échantillon afin de fournir un échantillon lysé, également appelé lysat,
- saisir le dispositif de filtration et de transfert 11 monté sur la barrette de support 3 à l'aide de la partie de manipulation 18,
- appliquer et maintenir une dépression entre -50 mbar et -980 mbar par rapport à la pression atmosphérique, préférentiellement à environ -600 mbar par rapport à la pression atmosphérique, dans le corps creux 12 du dispositif de filtration et de transfert 11,
- immerger au moins en partie la portion d'extrémité distale 12.2 du dispositif de filtration et de transfert 11 dans l'échantillon lysé contenu dans le puits d'échantillon 4,
- filtrer sous aspiration, par exemple pendant 2 minutes, au moins une partie de l'échantillon lysé à travers l'élément de filtration 13,
- capturer et accumuler des micro-organismes dans l'élément de filtration 13,
- immerger sous aspiration au moins en partie la portion d'extrémité distale 12.2 du dispositif de filtration et de transfert 11 dans le réactif de lavage contenu dans le puits de lavage 6.1,
- laver sous aspiration, dans le réactif de lavage contenu dans le puits de lavage 6.1, l'élément de filtration 13 et les micro-organismes capturés et accumulés,
- immerger sous aspiration au moins en partie la portion d'extrémité distale 12.2 du dispositif de filtration et de transfert 11 dans le réactif de lavage contenu dans le puits de lavage 6.2,
- laver sous aspiration, dans le réactif de lavage contenu dans le puits de lavage 6.2, l'élément de filtration 13 et les micro-organismes capturés et accumulés,
- remettre le corps creux 12 du dispositif de filtration et de transfert 11 à pression atmosphérique ou en légère surpression,
- remettre en suspension les micro-organismes dans une solution saline S1, par exemple à 0,45 %, contenue dans le puits de relargage 26 par exemple disposé sur le portoir 16 à proximité de la barrette de support 3, l'étape de remise en suspension comprenant les étapes suivantes :
   - immerger à pression atmosphérique ou en légère surpression (50 à 200 mbars) au moins en partie la portion d'extrémité distale 12.2 du dispositif de filtration et de transfert 11 dans la solution saline S1 contenue dans le puits de relargage 26,
   - mettre en contact de manière répétée, par exemple par un mouvement alternatif vertical du puits de relargage 26 et/ou du dispositif de filtration et de transfert 11, l'élément de filtration 13 avec une couche interne 26.1 en silicone réticulé, recouvrant au moins en partie la surface interne du puits de relargage 26, pendant une durée prédéterminée de manière à remettre en suspension les micro-organismes accumulés par l'élément de filtration 13 dans la solution saline S1 contenue dans le puits de relargage 26, l'étape de mise en contact peut par exemple consister à appliquer au dispositif de filtration et de transfert 11 une vibration comprise entre 0,1 et 200 Hz, par exemple entre 10 et 100 Hz, préférentiellement 50 Hz, pendant une durée prédéterminée, et par exemple pendant 30 secondes,
- optionnellement, réaliser cette mise en contact de manière répétée,
- mesurer la densité optique (D.O), calculer la dilution à effectuer et ajuster la D.O par pipetage d'un volume de solution saline présente en réserve dans le puits de lavage 6.3 en fonction du calcul effectué,
- prélever, dans le puits de relargage 26, une quantité prédéterminée de la solution saline S1 contenant les microorganismes, de préférence entre 100 et 300 µL et par exemple 145 ou 285 µL,
- introduire la solution saline prélevée dans un récipient d'analyse 27, par exemple disposé sur le portoir 16 à proximité du puits de relargage 26, contenant une quantité prédéterminée, par exemple entre 2 et 4 mL, préférentiellement 3 mL, de solution saline S2, par exemple à 0,45%,
- transférer le consommable contenu dans le récipient d'analyse 27, dans un analyseur d'antibiogramme,
- effectuer une analyse d'antibiogramme sur le consommable transféré.

Selon un mode de mise en oeuvre du deuxième procédé d'extraction, ce dernier comprend une étape consistant à réaliser une mesure de densité optique de la solution saline S1 contenue dans le puits de relargage 26.

Selon un mode de mise en oeuvre du deuxième procédé d'extraction, ce dernier comprend une étape consistant à ajuster à 0,5 McF la concentration de micro-organismes dans la solution saline S1 contenue dans le puits de relargage 26 par ajout de solution saline prélevée depuis le puits 6.3 contenant une réserve de solution saline 0,45%.

Selon un mode de mise en oeuvre du deuxième procédé d'extraction, le récipient d'analyse 27 pourrait être monté, par exemple de manière amovible, directement sur la barrette de support 3.

Selon une variante de mise en oeuvre du deuxième procédé d'extraction, l'étape de remise en suspension des micro-organismes pourrait consister à frotter l'élément de filtration 13 sur une grille en Nylon préalablement mouillée avec une solution saline à 0,45%.

Selon encore une autre variante de mise en oeuvre du deuxième procédé d'extraction, l'étape de remise en suspension pourrait comprendre les étapes suivantes :
- introduire au moins en partie le dispositif de filtration et de transfert 11 dans le puits de relargage 26,
- découper l'élément de filtration 13, par exemple à l'aide d'une pièce de découpe insérée dans le puits de relargage 26,
- libérer l'élément de filtration 13 dans la solution saline S1 contenue dans le puits de relargage 26,
- mettre en mouvement le puits de relargage 26 pendant une durée prédéterminée, par exemple durant 30 secondes à 1 minute, de manière à libérer les microorganismes accumulés dans l'élément de filtration 13 et à les remettre en suspension dans la solution saline S1 contenue dans le puits de relargage 26.

Les figures 9 et 10 représentent un système d'extraction 2 selon un troisième mode de réalisation qui diffère du premier mode de réalisation essentiellement en ce que le système d'extraction 2 comprend un organe d'obturation 28 déplaçable entre une position d'obturation dans laquelle l'organe d'obturation 28 obture le puits d'échantillon 4 et une position de libération dans laquelle l'organe d'obturation 28 libère au moins en partie le puits d'échantillon 4. L'organe d'obturation 28 peut par exemple être monté pivotant ou coulissant sur la barrette de support 3. En outre, le portoir 16 pourrait être configuré pour automatiquement déplacer l'organe d'obturation 28 dans la position d'obturation lors du positionnement de la barrette de support sur le portoir 16.

La barrette de support 3 pourrait comprendre additionnellement un dispositif de verrouillage configuré pour coopérer avec un portoir 16 de manière à verrouiller la barrette de support 3 sur le portoir 16. Le dispositif de verrouillage pourrait par exemple comporter (voir figure 2) un élément de verrouillage, tel qu'un ergot de verrouillage, configuré pour coopérer avec le portoir de manière à immobiliser la barrette de support 3 selon une direction Y d'un repère cartésien, et un élément de retenue, tel qu'une bille en acier, configuré pour coopérer avec un aimant permanent prévu sur le portoir 16 de manière à immobiliser la barrette de support 3 selon une direction Z du repère cartésien lors de l'insertion de la barrette dans le portoir. La bille en acier pourrait cependant être remplacée par exemple par un logement de retenue configuré pour recevoir un ergot de retenue prévu sur le portoir.

Comme il va de soi, l'invention ne se limite pas à la seule forme d'exécution de ce système d'extraction, décrite ci-dessus à titre d'exemple, elle en embrasse au contraire toutes les variantes de réalisation.

## Revendications

1. Système d'extraction (2) pour extraire des micro-organismes contenus dans un échantillon, le système d'extraction (2) comprenant :
- une barrette de support (3) comprenant :
- un puits d'échantillon (4) destiné à recevoir un échantillon contenant des micro-organismes, tel qu'une hémoculture positive,
- un puits de réactif (5) destiné à contenir un tampon de lyse sélective,
- au moins un puits de lavage (6.1) destiné à contenir un réactif de lavage, et
- au moins un logement de protection et de rétention (9, 9.1, 9.2),
- un élément de pipetage (10) monté de manière amovible sur la barrette de support (3) et s'étendant au moins en partie dans l'au moins un logement de protection et de rétention (9),
- un dispositif de filtration et de transfert (11) destiné à capturer et accumuler des micro-organismes contenus dans l'échantillon et à transférer les microorganismes capturés et accumulés, le dispositif de filtration et de transfert (11) étant monté de manière amovible sur la barrette de support (3) et s'étendant au moins en partie dans l'au moins un logement de protection et de rétention (9), le dispositif de filtration et de transfert (11) comprenant :
- un corps creux (12) ayant une forme allongée et comportant une portion d'extrémité proximale (12.1) et une portion d'extrémité distale (12.2), et
- un élément de filtration (13) fixé à la portion d'extrémité distale (12.2) du corps creux (12), l'élément de filtration ayant une forme cylindrique de diamètre adapté au diamètre d'un emplacement d'une plaque d'analyse MALDI-TOF MS, et présentant une partie distale plate ou hémisphérique..

2. Système d'extraction selon la revendication 1, dans lequel la portion d'extrémité distale (12.2) du corps creux (12) et l'élément de filtration (13) s'étendent dans l'au moins un logement de protection et de rétention (9).

3. Système d'extraction (2) selon la revendication 1 ou 2, dans lequel l'élément de pipetage (10) comporte une portion d'extrémité distale (10.1) s'étendant au moins en partie dans l'au moins un logement de protection et de rétention (9).

4. Système d'extraction (2) selon l'une quelconque des revendications 1 à 3, dans lequel le dispositif de filtration et de transfert (11) comporte un matériau absorbant disposé dans le corps creux.

5. Système d'extraction (2) selon l'une quelconque des revendications 1 à 4, dans lequel la barrette de support (3) comprend en outre un puits de relargage (26).

6. Système d'extraction (2) selon l'une quelconque des revendications 1 à 5, dans lequel l'au moins un puits de lavage (6.1) contient un réactif de lavage et le puits de réactif (5) contient un tampon de lyse sélective (L).

7. Système d'extraction (2) selon la revendication 6, dans lequel le puits de réactif (5) et l'au moins un puits de lavage (6.1) sont chacun obturés par un élément d'obturation (7).

8. Système d'extraction (2) selon l'une quelconque des revendications 1 à 7, lequel comprend en outre un organe d'obturation (28) déplaçable entre une position d'obturation dans laquelle l'organe d'obturation (28) obture le puits d'échantillon (4) et une position de libération dans laquelle l'organe d'obturation (28) libère au moins en partie le puits d'échantillon (4).

9. Procédé d'identification et/ou de caractérisation de micro-organismes contenus dans un échantillon, le procédé d'identification et/ou de caractérisation comprenant les étapes suivantes :
- prévoir un système d'extraction (2) selon l'une quelconque des revendications 1 à 8, dans lequel le puits de réactif (5) contient un tampon de lyse sélective et l'au moins un puits de lavage (6.1) contient un réactif de lavage,
- introduire une quantité prédéterminée d'un échantillon contenant des micro-organismes dans le puits d'échantillon (4),
- prélever une quantité prédéterminée du tampon de lyse sélective contenu dans le puits de réactif (5) à l'aide de l'élément de pipetage (10),
- introduire, dans le puits d'échantillon (4) et à l'aide de l'élément de pipetage (10), le tampon de lyse sélective prélevé,
- lyser des cellules et/ou des particules non micro-organiques contenues dans l'échantillon et/ou lyser des micro-organismes prédéterminés contenus dans l'échantillon de manière à fournir un échantillon lysé, - immerger au moins en partie le dispositif de filtration et de transfert (11) dans l'échantillon lysé contenu dans le puits d'échantillon (4),
- filtrer sous aspiration au moins une partie de l'échantillon lysé à travers l'élément de filtration (13),
- capturer et accumuler des micro-organismes dans l'élément de filtration (13),
- immerger au moins en partie le dispositif de filtration et de transfert (11) dans le réactif de lavage contenu dans l'au moins un puits de lavage (6.1),
- laver, dans le réactif de lavage contenu dans l'au moins un puits de lavage (6), l'élément de filtration (13) et les micro-organismes capturés et accumulés,
- transférer, à l'aide du dispositif de filtration et de transfert (11), les micro-organismes lavés sur un support d'analyse ou dans un récipient,
- analyser les micro-organismes transférés de manière à les identifier et/ou les caractériser.

10. Procédé selon la revendication 9, dans lequel l'étape de transfert comprend une étape consistant à remettre en suspension les micro-organismes lavés dans une solution saline (SI) contenue dans un puits de relargage (26).

11. Procédé selon la revendication 10, dans lequel l'étape de remise en suspension comprenant les étapes suivantes :
- immerger au moins en partie le dispositif de filtration et de transfert (11) dans la solution saline SI contenue dans le puits de relargage (26),
- mettre en contact de manière répétée l'élément de filtration (13) avec une couche interne en silicone réticulé (26.1) recouvrant au moins en partie la surface interne du puits de relargage (26) pendant une durée prédéterminée.

12. Procédé selon la revendication 11, lequel comprend en outre les étapes suivantes :
- réaliser une mesure de densité optique de la solution saline (SI) contenue dans le puits de relargage (26) et contenant les micro-organismes lavés,
- ajuster, par exemple à 0,5 McF, la concentration de micro-organismes dans la solution saline (SI) contenue dans le puits de relargage (26) par ajout de solution saline dans le puits de relargage (26),
- prélever, dans le puits de relargage (26), une quantité prédéterminée de la solution saline (SI) contenant les microorganismes, - introduire la solution saline prélevée (SI) dans un récipient d'analyse (27) contenant une quantité prédéterminée de solution saline (S2).

13. Procédé selon la revendication 12, dans lequel l'étape d'analyse comprend les étapes suivantes :
- transférer une quantité prédéterminée du consommable, contenu dans le récipient d'analyse (27) et contenant les micro-organismes transférés, dans un analyseur d'antibiogramme, et
- effectuer une analyse d'antibiogramme sur le consommable transféré.

14. Procédé selon la revendication 9, dans lequel l'étape de transfert comprend une étape consistant à déposer, à l'aide du dispositif de filtration et de transfert (11), au moins une partie des micro-organismes lavés sur un ou plusieurs emplacements prédéterminés (21) d'une plaque d'analyse MALDI-TOF MS (22), et dans lequel l'étape d'analyse comprend une étape consistant à effectuer une analyse en spectrométrie de masse de type MALDI-TOF des micro-organismes déposés sur ladite plaque MALDI-TOF MS (22).

## Patentansprüche

1. Extraktionssystem (2) zum Extrahieren von in einer Probe enthaltenen Mikroorganismen, wobei das Extraktionssystem (2) umfasst:
- einen Halterungssteg (3), umfassend:
- eine Probenvertiefung (4), die dazu bestimmt ist, eine Mikroorganismen enthaltende Probe wie etwa eine positive Blutkultur aufzunehmen,
- eine Reagensvertiefung (5), die dazu bestimmt ist, einen Puffer zur selektiven Lyse zu enthalten,
- mindestens eine Waschvertiefung (6.1), die dazu bestimmt ist, ein Waschreagens zu enthalten, und
- mindestens eine Schutz- und Halteaufnahme (9, 9.1, 9.2),
- ein Pipettierelement (10), das lösbar an dem Halterungssteg (3) angebracht ist und sich mindestens teilweise in der mindestens einen Schutz- und Halteaufnahme (9) erstreckt,
- eine Filtrations- und Transfervorrichtung (11), die dazu bestimmt ist, in der Probe enthaltene Mikroorganismen aufzufangen und zu sammeln und die aufgefangenen und gesammelten Mikroorganismen zu transferieren, wobei die Filtrations- und Transfervorrichtung (11) lösbar an dem Halterungssteg (3) angebracht ist und sich mindestens teilweise in der mindestens einen Schutz- und Halteaufnahme (9) erstreckt, wobei die Filtrations- und Transfervorrichtung (11) umfasst:
- einen hohlen Körper (12), der eine langgestreckte Form hat und einen proximalen Endabschnitt (12.1) und einen distalen Endabschnitt (12.2) beinhaltet, und
- ein Filtrationselement (13), das an dem distalen Endabschnitt (12.2) des hohlen Körpers (12) befestigt ist, wobei das Filtrationselement eine zylindrische Form mit einem Durchmesser hat, der an den Durchmesser eines Platzes einer MALDI-TOF MS Analyseplatte angepasst ist, und einen flachen oder halbkugelförmigen distalen Teil aufweist.

2. Extraktionssystem nach Anspruch 1, bei dem der distale Endabschnitt (12.2) des hohlen Körpers (12) und das Filtrationselement (13) sich in mindestens einer Schutz- und Halteaufnahme (9) erstrecken.

3. Extraktionssystem (2) nach Anspruch 1 oder 2, bei dem das Pipettierelement (10) einen distalen Endabschnitt (10.1) beinhaltet, der sich mindestens teilweise in der mindestens einen Schutz- und Halteaufnahme (9) erstreckt.

4. Extraktionssystem (2) nach einem der Ansprüche 1 bis 3, bei dem die Filtrations- und Transfervorrichtung (11) ein absorbierendes Material beinhaltet, das in dem hohlen Körper angeordnet ist.

5. Extraktionssystem (2) nach einem der Ansprüche 1 bis 4, bei dem der Halterungssteg (3) ferner eine Aussalzvertiefung (26) umfasst.

6. Extraktionssystem (2) nach einem der Ansprüche 1 bis 5, bei dem die mindestens eine Waschvertiefung (6.1) ein Waschreagens enthält und die Reagensvertiefung (5) einen Puffer zur selektiven Lyse (L) enthält.

7. Extraktionssystem (2) nach Anspruch 6, bei dem die Reagensvertiefung (5) und die mindestens eine Waschvertiefung (6.1) jeweils durch ein Verschlusselement (7) verschlossen werden.

8. Extraktionssystem (2) nach einem der Ansprüche 1 bis 7, das ferner ein Verschlussorgan (28) umfasst, das zwischen einer Verschlussposition, in der das Verschlussorgan (28) die Probenvertiefung (4) verschließt, und einer Freigabeposition, in der das Verschlussorgan (28) die Probenvertiefung (4) mindestens teilweise freigibt, verlagerbar ist.

9. Verfahren zum Identifizieren und/oder Charakterisieren von in einer Probe enthaltenen Mikroorganismen, wobei das Verfahren zum Identifizieren und/oder Charakterisieren die folgenden Schritte umfasst:
- Vorsehen eines Extraktionssystems (2) nach einem der Ansprüche 1 bis 8, bei dem die Reagensvertiefung (5) einen Puffer zur selektiven Lyse enthält und die mindestens eine Waschvertiefung (6.1) ein Waschreagens enthält,
- Einführen einer vorbestimmten Menge einer Mikroorganismen enthaltenden Probe in die Probenvertiefung (4),
- Entnehmen einer vorbestimmten Menge des in der Reagensvertiefung (5) enthaltenen Puffers zur selektiven Lyse mithilfe des Pipettierelements (10),
- Einführen des entnommenen Puffers zur selektiven Lyse in die Probenvertiefung (4) und mithilfe des Pipettierelements (10),
- Lysieren von in der Probe enthaltenen nicht mikroorganischen Zellen und/oder Partikeln und/oder Lysieren von in der Probe enthaltenen vorbestimmten Mikroorganismen, so dass eine lysierte Probe bereitgestellt wird,
- mindestens teilweises Eintauchen der Filtrations- und Transfervorrichtung (11) in die in der Probenvertiefung (4) enthaltene lysierte Probe,
- Filtrieren unter Absaugung mindestens eines Teils der lysierten Probe durch das Filtrationselement (13) hindurch,
- Auffangen und Sammeln der Mikroorganismen in dem Filtrationselement (13),
- mindestens teilweises Eintauchen der Filtrations- und Transfervorrichtung (11) in das in der mindestens einen Waschvertiefung (6.1) enthaltene Waschreagens,
- Waschen, in dem in der mindestens einen Waschvertiefung (6) enthaltenen Waschreagens, des Filtrationselements (13) und der aufgefangenen und gesammelten Mikroorganismen,
- Transferieren, mithilfe der Filtrations- und Transfervorrichtung (11), der gewaschenen Mikroorganismen auf einen Analyseträger oder in ein Behältnis,
- Analysieren der transferierten Mikroorganismen, so dass sie identifiziert und/oder charakterisiert werden.

10. Verfahren nach Anspruch 9, bei dem der Transferschritt einen Schritt umfasst, der darin besteht, die gewaschenen Mikroorganismen in einer in einer Aussalzvertiefung (26) enthaltenen Salzlösung (Sl) zu resuspendieren.

11. Verfahren nach Anspruch 10, bei dem der Schritt des Resuspendierens die folgenden Schritte umfasst:
- mindestens teilweises Eintauchen der Filtrations- und Transfervorrichtung (11) in die in der Aussalzvertiefung (26) enthaltene Salzlösung Sl,
- wiederholtes Kontaktieren des Filtrationselements (13) mit einer inneren Schicht aus vernetztem Silikon (26.1), welche die innere Oberfläche der Aussalzvertiefung (26) mindestens teilweise bedeckt, während einer vorbestimmten Dauer.

12. Verfahren nach Anspruch 11, das ferner die folgenden Schritte umfasst:
- Ausführen einer Messung der optischen Dichte der Salzlösung (Sl), die in der Aussalzvertiefung (26) enthalten ist und die gewaschenen Mikroorganismen enthält,
- Einstellen, zum Beispiel auf 0,5 McF, der Mikroorganismenkonzentration in der in der Aussalzvertiefung (26) enthaltenen Salzlösung (Sl) durch Zugeben von Salzlösung in die Aussalzvertiefung (26),
- Entnehmen, in der Aussalzvertiefung (26), einer vorbestimmten Menge der die Mikroorganismen enthaltenden Salzlösung (Sl),
- Einführen der entnommenen Salzlösung (Sl) in ein Analysebehältnis (27), das eine vorbestimmte Menge Salzlösung (S2) enthält.

13. Verfahren nach Anspruch 12, bei dem der Analyseschritt die folgenden Schritte umfasst:
- Transferieren einer vorbestimmten Menge des Verbrauchsmaterials, das in dem Analysebehältnis (27) enthalten ist und die transferierten Mikroorganismen enthält, in einen Antibiogramm-Analysator und
- Durchführen einer Antibiogramm-Analyse an dem transferierten Verbrauchsmaterial.

14. Verfahren nach Anspruch 9, bei dem der Schritt des Transferierens einen Schritt umfasst, der darin besteht, mithilfe der Filtrations- und Transfervorrichtung (11) mindestens einen Teil der gewaschenen Mikroorganismen auf einen oder mehrere vorbestimmte Plätze (21) einer MALDI-TOF MS Analyseplatte (22) abzusetzen, und bei dem der Analyseschritt einen Schritt umfasst, der darin besteht eine Massenspektrometrie-Analyse vom Typ MALDI-TOF der auf der MALDI-TOF MS Analyseplatte (22) abgesetzten Mikroorganismen durchzuführen.

## Claims

1. Extraction system (2) for extracting microorganisms contained in a sample, said extraction system (2) comprising:
- a support bar (3) comprising:
- a sample well (4) intended to receive a sample containing microorganisms, such as a positive blood culture,
- a reagent well (5) intended to contain a selective lysis buffer,
- at least one washing well (6.1) intended to contain a washing reagent, and
- at least one protection and retention housing (9, 9.1, 9.2),
- a pipetting element (10) removably mounted on the support bar (3) and extending at least partially into the at least one protection and retention housing (9),
- a filtering and transfer device (11) intended to capture and accumulate microorganisms contained in the sample and transfer the captured and accumulated microorganisms, the filtering and transfer device (11) being removably mounted on the support bar (3) and extending at least partially into the at least one protection and retention housing (9), said filtering and transfer device (11) comprising:
- a hollow body (12) having an elongated shape and comprising a proximal end portion (12.1) and a distal end portion (12.2), and
- a filtering element (13) fixed to the distal end portion (12.2) of the hollow body (12), the filtering element having a cylindrical shape with a diameter adapted to the diameter of a spot of a MALDI-TOF MS analysis plate, and having a flat or hemispherical distal portion.

2. Extraction system according to Claim 1, in which the distal end portion (12.2) of the hollow body (12) and the filtering element (13) extend into the at least one protection and retention housing (9).

3. Extraction system (2) according to Claim 1 or 2, in which the pipetting element (10) comprises a distal end portion (10.1) extending at least partially into the at least one protection and retention housing (9).

4. Extraction system (2) according to any one of Claims 1 to 3, in which the filtering and transfer device (11) comprises an absorbent material arranged in the hollow body.

5. Extraction system (2) according to any one of Claims 1 to 4, in which the support bar (3) further comprises a release well (26).

6. Extraction system (2) according to any one of Claims 1 to 5, in which the at least one washing well (6.1) contains a washing reagent and the reagent well (5) contains a selective lysis buffer (L).

7. Extraction system (2) according to Claim 6, in which the reagent well (5) and the at least one washing well (6.1) are each sealed by a sealing element (7).

8. Extraction system (2) according to any one of Claims 1 to 7, which further comprises a sealing device (28) movable between a sealing position in which the sealing device (28) seals the sample well (4) and a release position in which the sealing device (28) releases the sample well (4) at least partly.

9. Method for identifying and/or characterizing microorganisms contained in a sample, said identifying and/or characterizing method comprising the following steps:
- providing an extraction system (2) according to any one of Claims 1 to 8, in which the reagent well (5) contains a selective lysis buffer and the at least one washing well (6.1) contains a washing reagent,
- introducing a predetermined amount of a sample containing microorganisms in the sample well (4),
- withdrawing a predetermined amount of the selective lysis buffer contained in the reagent well (5) by means of the pipetting element (10),
- placing the withdrawn selective lysis buffer in the sample well (4) by means of the pipetting element (10),
- lysing cells and/or particles that are not micro-organic contained in the sample and/or lysing predetermined microorganisms contained in the sample so as to supply a lysed sample,
- at least partially immersing the filtering and transfer device (11) in the lysed sample contained in the sample well (4),
- filtering at least part of the lysed sample through the filtering element (13) under aspiration,
- capturing and accumulating microorganisms in the filtering element (13),
- at least partially immersing the filtering and transfer device (11) in the washing reagent contained in the at least one washing well (6.1),
- washing the filtering element (13) and the captured and accumulated microorganisms in the washing reagent contained in the at least one washing well (6),
- by means of the filtering and transfer device (11), transferring the washed microorganisms onto an analysis support or into a receiver,
- analyzing the transferred microorganisms so as to identify them and/or characterize them.

10. Method according to Claim 9, in which the transfer step comprises a step consisting of resuspending the washed microorganisms in a saline solution (Sl) contained in a release well (26).

11. Method according to Claim 10, in which the resuspending step comprises the following steps:
- at least partially immersing the filtering and transfer device (11) in the saline solution (Sl) contained in the release well (26),
- repeatedly contacting the filtering element (13) with an inner layer of crosslinked silicone (26.1) at least partly covering the inside surface of the release well (26) for a predetermined time.

12. Method according to Claim 11, which further comprises the following steps:
- measuring the optical density of the saline solution (Sl) contained in the release well (26) and containing the washed microorganisms,
- adjusting, for example to 0.5 µF, the concentration of microorganisms in the saline solution (Sl) contained in the release well (26) by adding saline solution to the release well (26),
- withdrawing, from the release well (26), a predetermined amount of the saline solution (Sl) containing the microorganisms,
- introducing the withdrawn saline solution (Sl) into an analysis container (27) containing a predetermined amount of saline solution (S2).

13. Method according to Claim 12, in which the analysis step comprises the following steps:
- transferring a predetermined amount of the consumable contained in the analysis container (27) and containing the transferred microorganisms, into an antibiogram analyzer, and
- performing an antibiogram analysis on the consumable transferred.

14. Method according to Claim 9, in which the transfer step comprises a step consisting of depositing, by means of the filtering and transfer device (11), at least one part of the washed microorganisms onto one or more predetermined spots (21) of a MALDI-TOF MS analysis plate (22), and in which the analysis step comprises a step consisting of performing a mass spectrometry analysis of the MALDI-TOF type on the microorganisms deposited on said MALDI-TOF MS plate (22) .
